(19) Europäisches Patentamt
European Patent Office
Office européen des brevets

(11) EP 1 563 136 B1

(12) **EUROPÄISCHE PATENTSCHRIFT**

(45) Veröffentlichungstag und Bekanntmachung des Hinweises auf die Patenterteilung:
**14.05.2008 Patentblatt 2008/20**

(21) Anmeldenummer: **03810454.3**

(22) Anmeldetag: **31.10.2003**

(51) Int Cl.:
***D06M 15/643*** *(2006.01)* ***C08G 77/388*** *(2006.01)*
***C08G 77/452*** *(2006.01)*

(86) Internationale Anmeldenummer:
**PCT/EP2003/050773**

(87) Internationale Veröffentlichungsnummer:
**WO 2004/042136 (21.05.2004 Gazette 2004/21)**

(54) **LINEARE POLYAMINO- UND/ODER POLYAMMONIUM-POLYSILOXANCOPOLYMERE II**

LINEAR POLYAMINO AND/OR POLYAMMONIUM POLYSILOXANE COPOLYMERS II

COPOLYMERES POLYSILOXANE POLYAMINO ET/OU POLYAMMONIUM II LINEAIRES

(84) Benannte Vertragsstaaten:
**AT BE BG CH CY CZ DE DK EE ES FI FR GB GR HU IE IT LI LU MC NL PT RO SE SI SK TR**

(30) Priorität: **04.11.2002 DE 10251524**

(43) Veröffentlichungstag der Anmeldung:
**17.08.2005 Patentblatt 2005/33**

(73) Patentinhaber: **Momentive Performance Materials GmbH**
**51368 Leverkusen (DE)**

(72) Erfinder:
- **LANGE, Horst**
  **44879 Bochum (DE)**
- **WITOSSEK, Anita**
  **40764 Langenfeld (DE)**
- **WAGNER, Roland**
  **53757 Bonn (DE)**
- **STACHULLA, Karl-Heinz**
  **51370 Leverkusen (DE)**
- **GRAYDON, Andrew Russell**
  **Tyne and Wear NE8 1TP (GB)**
- **HARTSHORN, Richard, Timothy**
  **Cincinnati, OH 45208 (US)**
- **BOUTIQUE, Jean-Pol**
  **B-5030 Gembloux (BE)**
- **DELPLANQUE, Patrick, Firmin, August**
  **B-9270 Laarne (BE)**
- **JOHNSTON, James, Pyott**
  **B-1785 Merchtem (BE)**
- **SOCKEL, Karl-Heinz**
  **51373 Leverkusen (DE)**

(56) Entgegenhaltungen:
**WO-A-02/10256** **WO-A-02/10259**
**DE-A- 10 036 699** **US-A- 4 833 225**
**US-A- 5 807 956**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 563 136 B1

## Beschreibung

Die Erfindung betrifft lineare Polyamino- und/oder Polyammonium-Polysiloxancopolymere, insbesondere hydrophile polyquaternäre Polysiloxancopolymere und deren Verwendung als waschbeständige hydrophile Weichmacher.

Aminogruppen-enthaltende Polysiloxane sind als textile Weichmacher bekannt (EP 441530). Die Einführung von durch Ethylenoxid-/Propylenoxideinheiten modifizierten Aminostrukturen als Seitenketten bewirkt eine Verbesserung des Effekts (US 5,591,880, US 5,650,529). Die Alkylenoxideinheiten erlauben hierbei die gezielte Einstellung der hydrophilen-hydrophoben Balance. Nachteilig ist vom synthetischen Standpunkt aus die in die Synthesestrategie eingeschlossene schwierige Veresterung von Aminoalkoholen mit siloxangebundenen Carbonsäuregruppen und bezüglich der weichmachenden Eigenschaften die generelle Kammstruktur der Produkte.

Zur Beseitigung dieser Nachteile ist vorgeschlagen worden, α,ω-epoxymodifizierte Siloxane mit α,ω-aminofünktionalisierten Alkylenoxiden umzusetzen, und diese Produkte als hydrophile Weichmacher einzusetzen (US 5,807,956, US 5,981,681).

Zur Verbesserung der Substantivität sind Versuche unternommen worden, quartäre Ammoniumgruppen in alkylenoxidmodifizierte Siloxane einzuführen.

Verzweigte alkylenoxidmodifizierte Polysiloxanquats ("Polysiloxanquats" sind Polydiorganosiloxan-Polyalkylammonium-Verbindungen) sind aus α,ω-OH terminierten Polysiloxanen und Trialkoxysilanen durch Kondensation synthetisiert worden. Die quartäre Ammoniumstruktur wird über das Silan eingebracht, wobei das quartäre Stickstoffatom durch Alkylenoxideinheiten substituiert ist (US 5,602,224).

Streng kammartige alkylenoxidmodifizierte Polysiloxanquats sind ebenfalls beschrieben worden (US 5,098,979). Die Hydroxylgruppen von kammartig substituierten Polyethersiloxanen werden mit Epichlorhydrin in die entsprechenden Chlorhydrinderivate überführt. Anschließend erfolgt eine Quaternierung mit tertiären Aminen. Aus diesem Grund heraus sind die Hydroxylgruppen kammartig substituierter Polyethersiloxane alternativ mit Chloressigsäure verestert worden.

Durch die Carbonylaktivierung kann die abschließende Quaternierung erleichtert vollzogen werden (US 5,153,294, US 5,166,297).

In US 6,242,554 werden α,ω-difunktionelle Siloxanderivate beschrieben, die jeweils über eine separate quartäre Ammonium- und Alkylenoxideinheit verfügen. Diese Materialien zeichnen sich durch eine verbesserte Kompatibilität zu polaren Umgebungen aus.

Die Reaktion von α,ω-Diepoxiden mit tertiären Aminen in Gegenwart von Säuren liefert α,ω-diquartäre Siloxane, welche zu Haarpflegezwecken eingesetzt werden können (DE-PS 37 19 086). Neben tetraalkylsubstituierten quartären Ammoniumstrukturen werden auch aromatische Imidazoliniumderivate beansprucht.

Eine Verringerung der Auswaschbarkeit aus Haaren kann erzielt werden, wenn die α,ω-Diepoxide mit ditertiären Aminen in Gegenwart von Säuren zu langkettigen polyquartären Polysiloxanen umgesetzt werden (EP 282720). Aromatische quartäre Ammoniumstrukturen werden nicht offenbart. Derartige Derivate werden in US 6240929 behandelt. In einem ersten Schritt werden hierzu aus Imidazol und geeigneten difunktionellen Alkylierungsagenzien zwei Imidazoleinheiten aufweisende Diamine synthetisiert, welche nachfolgend in einer zur EP 282720 analogen Weise in polyquaternäre Polysiloxane überführt werden. Auf diese Weise hergestellte kationische Verbindungen sollen eine weiter erhöhte Kompatibilität gegenüber den in kosmetischen Formulierungen vorhandenen anionischen Tensiden besitzen.

Allerdings bezieht sich die Auswaschbeständigkeit aus Haaren auf den kurzzeitigen Angriff von vornehmlich Wasser und sehr milden, die Haut nicht irritierenden Tensiden, während waschbeständige, hydrophile Weichmacher für Textilien dem Angriff konzentrierter Tensidlösungen mit hohem Fett- und Schmutzlösevermögen zu widerstehen haben. Erschwerend kommt hinzu, daß moderne Waschmittel stark alkalische Komplexbildner, oxydativ wirkende Bleichmittel und komplexe Enzymsysteme enthalten und die Fasern der Einwirkung oftmals über Stunden bei erhöhten Temperaturen ausgesetzt sind.

Aus der WO 02/10259 sind polyquaternäre Polysiloxanverbindungen bekannt, in denen zusätzlich hydrophile Einheiten (EO-Einheiten) eingebaut sind sowie die Anordnung und Sequenzfolge der Quateinheiten zu hydrophilen Einheiten derart verändert werden kann, dass in der Folge ein besserer hydrophiler Weichgriff ohne Verlust der Substantivität auf z.B. Textilien ( Baumwolle, Polyester ) erzielbar wird.

Weitere Ansätze zur Verbesserung der Kompatibilität mit anionischen Tensidsystemen bzw. der Effizienz der Siloxanabscheidung auf Oberflächen zielen ab auf die Verwendung größerer Mengen kationischer Tenside (WO 00/71806 und WO 00/71807) oder die Nutzung kationischer Polysaccharidderivate (J.V.Gruber et. al., Colloids and Surfaces B: Biointerfaces 19 (2000) 127 - 135) in Mischungen mit Polydimethylsiloxanen.

Hoch geladene, sehr hydrophile synthetische Polykationics sind ebenfalls in der Lage, die Kompatibilität mit anionischen Tensidsystemen zu verbessern (US 6,211,139) oder in Gegenwart von Lösungen anionischer Tenside mit Fasern zu assoziieren (WO 99/14300). In der letztgenannten Schrift werden u.a. Polyimidazoliniumderivate beschrieben.

Keiner der behandelten Vorschläge stellt eine befriedigende Lösung für das Problem dar, den durch Silicone möglichen weichen Griff und die ausgeprägte Hydrophilie nach Erstausrüstung eines Textilmaterials auch dann zu

erhalten, wenn dieses dem Angriff aggressiver Detergenzienformulierungen im Verlauf wiederholter Waschprozesse bei gegebenenfalls erhöhter Temperatur ausgesetzt wird.

Ein grundsätzlich anderer Ansatz wird in DE-OS 32 36 466 beschrieben. Die Umsetzung von OH-terminierten Siloxanen mit quartäre Ammoniumstrukturen enthaltenden Alkoxysilanen liefert reaktive Zwischenprodukte, die mit geeigneten Vemetzungsagenzien, wie Trialkoxysilanen, auf der Faseroberfläche zu waschbeständigen Schichten vernetzen sollen. Entscheidender Nachteil dieses Ansatzes ist, daß die über Stunden notwendige Stabilität eines wässrigen Ausrüstungsbades nicht garantiert werden kann und unvorhergesehene Vernetzungsreaktionen im Bad bereits vor der Textilausrüstung auftreten können.

Aus der WO 02/10257 sind Polysiloxan-Verbindungen mit quaternären Ammoniumgruppen bekannt, die aus Diaminen, Polydiorganosiloxangruppenenthaltenden Diepoxiden und Di(halogenalkyl)-esterpolyether-Verbindungen aufgebaut werden. Diese enthalten jedoch herstellungsbedingt einen gewissen Anteil von gerade unter alkalischen Bedingungen hydrolyseempfindlichen Estergruppen als wesentlichen Bestandteil. Außerdem weisen die dort beschriebenen Polysiloxan-Verbindungen ein starres Verhältnis zwischen weichmachenden Polydiorganosiloxanblöcken und hydrophilen Blöcken auf. Die Eigenschaften dieser Polysiloxan-Verbindungen lassen sich somit nicht immer auf bestimmte Anforderungen maßschneidern. So ist für bestimmte Anwendungen die Hydrophilie dieser Polysiloxan-Verbindungen nicht immer zufriedenstellend, während in anderen

Anwendungen der Weichgriff oder die Substantivität zu wünschen übrig lassen.

Keine der zitierten Lösungen lehrt, wie eine weitere Erhöhung der Hydrophilie und der Substantivität unter Beibehaltung des Weichgriffes erreicht werden kann, oder wie sich insbesondere diese Eigenschaften für bestimmte Anwendungen quasi maßschneidern lassen.

Es ist somit Aufgabe der. Erfindung, lineare Polysiloxancopolymere, deren Herstellung und deren Verwendung als waschbeständige hydrophile Weichmacher bereitzustellen, wobei die linearen Polysiloxancopolymere den Textilien nach entsprechender Applikation einen silicontypischen weichen Griff und eine ausgeprägte Hydrophilie verleihen und dieses Eigenschaftsbild auch nach Einwirkung von Detergenzienformulierungen während wiederholter Waschprozesse bei gegebenenfalls erhöhter Temperatur nicht verloren geht. Es ist eine weitere Aufgabe der Erfindung, die Verwendung dieser linearen Polysiloxancopolymere als separate Weichmacher nach der Wäsche von Fasern und/oder Textilien bzw. als Weichmacher in der Wäsche mit auf nichtionogenen oder anionischen/nichtionogenen Tensiden beruhenden Formulierungen bereitzustellen. Ferner sollen die lineare Polysiloxancopolymere Textilverknitterungen verhindern bzw. rückgängig machen. Schließlich besteht die Aufgabe der vorliegenden Erfindung darin ein lineares Polysiloxancopolymer bereitzustellen, dessen Eigenschaften hinsichtlich Weichgriff, Substantivität, Hydrophilie oder dergleichen sich für eine jeweilige Anwendung in einfacher Weise, maßschneidern lässt.

Die vorliegende Erfindung stellt somit lineare Polyamino- und/oder Polyammonium-Polysiloxancopolymere mit der Wiederholungseinheit:

$$-[Q-V]- \qquad (I)$$

bereit, wie im Anspruch 1 definiert.

Q wird aus der Gruppe ausgewählt, die besteht aus:

einer Ammoniumeinheit der Formel:

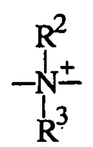

einer quatemierten Imidazoleinheit der Struktur

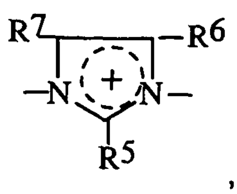

,

einer quaternierten Pyrazoleinheit der Struktur

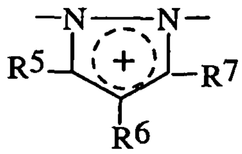

einer zweifach quatemierten Piperazineinheit der Struktur

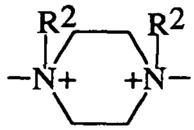

,

einer monoquaternierten Piperazineinheit der Struktur

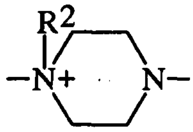

,

einer monoquatemierten Piperazineinheit der Struktur

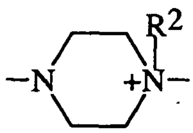

,

einer monoquatemierten Einheit der Struktur

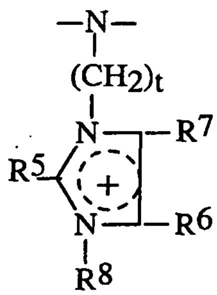

,

worin

t von 2 bis 10 ist,
$R^2$ wie oben definiert ist, und die Bedeutung von $R^2$ von der Bedeutung der obigen Gruppe $R^2$ gleich oder verschieden

sein kann,
$R^3$ die Bedeutung von $R^2$ aufweist, wobei $R^2$ und $R^3$ gleich oder verschieden sein können, oder
$R^2$ und $R^3$ gemeinsam mit dem positiv geladenen Stickstoffatom einen fünf- bis siebengliedrigen Heterocyclus bilden, der gegebenenfalls zusätzlich ein oder mehrere Stickstoff-, Sauerstoff- und/oder Schwefelatome aufweisen kann,
$R^5$, $R^6$, $R^7$ gleich oder verschieden sein können und aus der Gruppe ausgewählt werden, die besteht aus: H, Halogen, Hydroxylgruppe, Nitrogruppe, Cyanogruppe, Thiolgruppe, Carboxylgruppe, Alkylgruppe, Monohydroxyalkylgruppe, Polyhydroxyalkylgruppe, Thioalkylgruppe, Cyanoalkylgruppe, Alkoxygruppe, Acylgruppe, Acetyloxygruppe, Cycloalkylgruppe, Arylgruppe, Alkylarylgruppe, und Gruppen des Typs $NHR^W$, in denen $R^W$ H, Alkylgruppe, Monohydroxyalkylgruppe, Polyhydroxyalkylgruppe, Acetylgruppe, Ureidogruppe bedeuten, und jeweils zwei der benachbarten Reste $R^5$, $R^6$ und $R^7$ mit den sie an den Heterocyclus bindenden Kohlenstoffatomen aromatische Fünf- bis Siebenringe bilden können, und
$R^8$ die Bedeutung von $R^2$ aufweist, wobei $R^8$ und $R^2$ gleich oder verschieden sein können.

In einer weiteren bevorzugten Ausführungsform der vorliegenden Erfindung stellt $V^2$ eine Gruppe der Formel

$$-V^{2*}-Z^2-V^{2*}-$$

dar, worin $Z^2$ wie oben definiert ist und $V^{2*}$ einen zweiwertigen geradkettigen, cyclischen oder verzweigten, gesättigten, ungesättigten oder aromatischen Kohlenwasserstoffrest mit bis zu 40 Kohlenstoffatomen darstellt, der gegebenenfalls eine oder mehrere Gruppen, ausgewählt aus -O-, -CONH-, $-CONR^2-$, worin $R^2$ wie oben definiert ist, -C(O)- und -C(S)- enthalten kann, und der Rest $V^{2*}$ gegebenenfalls durch eine oder mehrere Hydroxylgruppen substituiert sein kann.

Bevorzugt enthält auch $V^2$ bzw. $V^{2*}$ keine Estergruppierungen -C(O)-O- bzw. -O-C(O)-.

Im Falle, dass Q einen dreiwertigen Rest der Formeln

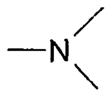
oder
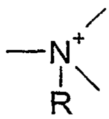

darstellt, dienen diese Reste nicht der Verzweigung der Polysiloxan-Copolymere sondern diese Reste sind ausschließlich mit dreiwertigen Resten $V^1$ oder $V^2$ verbunden, wobei cyclische Strukturen ausgebildet werden, die Bestandteil der linearen Hauptkette sind, wie z.B. ein Strukturelement der Formel

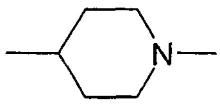
.

Gleichfalls dienen die dreiwertigen Reste $V^1$ bzw. $V^2$ nicht der Verzweigung der linearen Polysiloxan-Copolymere.

In der vorstehend erwähnten Ausführungsform weist das erfindungsgemäße lineare Polysiloxancopolymer die folgenden Wiederholungseinheiten auf:

$$-[V^{2*}-Z^2-V^{2*}-Q]- \text{ und } -[V^1-Q]-.$$

Das molare Verhältnis der Wiederholungseinheiten $-[V^{2*}-Z^2-V^{2*}-Q]-$ zu -[V'-Q]- entspricht dem Verhältnis $V^2/V^1 \neq 1$.

Aufgrund dieser molaren Verhältnisse enthalten die erfindungsgemäßen linearen Polysiloxancopolymere zwingend Blöcke, die mehr als eine $-[V^1-Q]$-Einheit bzw. $[V^2-Q]$-Einheit, miteinander verknüpft enthalten.

Wie weiter unten im Zusammenhang mit dem erfindungsgemäßen Verfahren zur Herstellung der lineare Polysiloxancopolymere der Erfindung noch ausführlich erläutert wird, können die blockartigen Sequenzen, die mehr als eine $-[V^1-Q]$-Einheit miteinander verknüpft aufweisen bei denen also $V^2/V^1 < 1$ ist, je nach Herstellweise regelmäßig mit den $V^2$-Q-Einheiten oder unregelmäßig mit den $V^2$-Q-Einheiten verbunden werden.
Dies meint folgendes:

Bei der regelmäßigen Verbindung, bei der beispielsweise ein der Gruppe $-Q-[V^1-Q]_x$-entsprechendes Präpolymer mit $V^2$ entsprechenden Monomer-Einheiten im molaren Verhältnis 1:1 umgesetzt wird, lassen sich die linearen Polysiloxancopolymere wie folgt darstellen:

$$-\{V^2-Q-[V^1-Q]_x-\}-.$$

x kann dabei bevorzugt 1,01 bis 2000 sein und ist der Mittelwert. Die durch die Formel $-\{V^2-Q-[V^1-Q]_x-\}-$ dargestellten linearen Polysiloxancopolymere sind dadurch gekennzeichnet, dass sie im wesentlichen keine miteinander verknüpften $-V^2-Q$-Einheiten aufweisen, oder mit anderen Worten, sind zwei $-V^2-Q$-Einheiten stets durch mindestens eine $-V^1-Q$-Einheit unterbrochen.

Ist $V^2/V^1 > 1$ so ist x in der obigen Formel bevorzugt etwa 0,001 bis 0,99. In diesem Fall enthalten die linearen Polysiloxancopolymere mindestens eine miteinander verknüpfte $-V^2-Q-$ Einheit, oder mit anderen Worten, sind zwei $V^1-Q$-Einheiten stets durch mindestens eine $V^2-Q-$ Einheit unterbrochen.

Bei der unregelmäßigen Verbindung, bei der beispielsweise Q-Einheiten entsprechende Monomere mit $V^1$ entsprechenden Monomer-Einheiten und $V^2$ entsprechenden Monomer-Einheiten im Verhältnis $Q/(V^1 + V^2)$, mit $V^2/V^1 \neq 1$, von 1:1 umgesetzt wird, lassen sich die linearen Polysiloxancopolymere wie folgt darstellen:

$$-Q-(V^1,V^2)-,$$

worin das Verhältnis $V^2/V^1 \neq 1$ ist. Dabei sind die Gruppen $V^1$ und $V^2$ statistisch über die Copolymerkette verteilt. Im Unterschied zu dem durch die regelmäßige Verbindung hergestellten linearen Polysiloxancopolymere kann dieses Copolymer auch benachbarte $-Q-V^2-$ bzw. $-Q-V^1$-Einheiten aufweisen.

In einer weiteren bevorzugten Ausführungsform der vorliegenden Erfindung wird die Gruppe $V^1$ ausgewählt aus zweiwertigen, geradkettigen, cyclischen oder verzweigten, gesättigten, ungesättigten oder aromatischen Kohlenwasserstoffresten mit bis zu 400 Kohlenstoffatomen, die gegebenenfalls eine oder mehrere Gruppen, ausgewählt aus -O-, -CONH-, $-CONR^2-$, worin $R^2$ wie oben definiert ist, -C(O)-, -C(S)- und $-Z^1-$ enthalten kann, worin $-Z^1-$ eine Gruppe der Formel

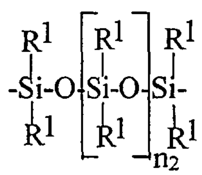

ist, worin

$R^1$ $C_1$ bis $C_3$ Alkyl, Fluor($C_3$-$C_6$)alkyl oder $C_6$-Aryl ist, und $n_2$ wie oben definiert ist. In einer weiteren bevorzugten Ausführungsform der vorliegenden Erfindung wird die Gruppe Q ausgewählt aus:

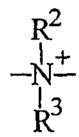

einer quaternierten Imidazoleinheit der Struktur

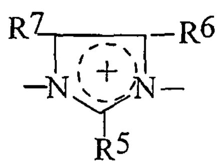

,

einer quaternierten Pyrazoleinheit der Struktur

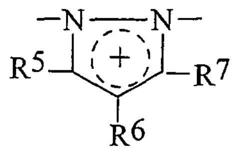

einer zweifach quaternierten Piperazineinheit der Struktur

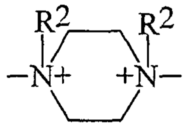

,

einer monoquaternierten Piperazineinheit der Struktur

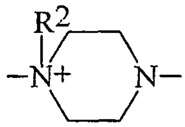

,

einer monoquaternierten Piperazineinheit der Struktur

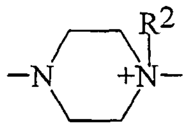

,

einer monoquaternierten Einheit der Struktur

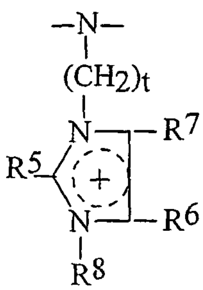

,

worin $R^2$, $R^3$, $R^4$, $R^5$, $R^6$, $R^7$ und $R^8$ wie oben definiert sind.

In einer weiteren bevorzugten Ausführungsform der vorliegenden Erfindung, insbesondere in Anwendungen, bei denen eine erhöhte Hydrophilie der erfindungsgemäßen linearen Polysiloxancopolymere im Vordergrund steht, erfüllt das molare Verhältnis $V^2/V^1$ die Beziehung

$$V^2/V^1 < 1$$

bevorzugter die Beziehung

$$0{,}0005 < V^2/V^1 < 0{,}9$$

noch bevorzugter die Beziehung

$$0{,}005 < V^2/V^1 < 0{,}8,$$

noch bevorzugter die Beziehung

$$0{,}01 < V^2/V^1 < 0{,}5.$$

In einer weiteren bevorzugten Ausführungsform der vorliegenden Erfindung, insbesondere in Anwendungen, bei denen ein erhöhter Weichgriff bei gleichbleibender Substantivität der erfindungsgemäßen lineare Polysiloxancopolymere im Vordergrund steht, wie zum Beispiel bei bestimmten Textilausrüstungen, erfüllt das molare Verhältnis $V^2/V^1$ bevorzugt die Beziehung

$$V^2/V^1 > 1$$

bevorzugter die Beziehung

$$1 < V^2/V^1 < 1000,$$

noch bevorzugter die Beziehung

$$1{,}1 < V^2/V^1 < 100,$$

noch bevorzugter die Beziehung

$$2 < V^2/V^1 < 20.$$

Bevorzugt sind:

$R^1$ = $C_1$ bis $C_{18}$ Alkyl, insbesondere Methyl, Ethyl, Perfluoralkylethylen, wie Trifluorpropyl und Phenyl,

$n_1$ = 20 bis 400, besonders bevorzugt 20 bis 300, speziell 20 bis 200. In einer weiteren bevorzugten Ausführungsform ist $n_1$ zwischen 20 und 50 oder zwischen 80 und 200. Die Zahl $n_1$ ist die mittlere Polymerisationsgrad der Diorganosiloxy-Einheiten in der Gruppe $Z^2$.

$n_2$ = 0 bis 15, besonders bevorzugt 0 bis 10, speziell 0 bis 5, spezieller 0. Die Zahl $n_2$ ist die mittlere Polymerisationsgrad aus Mn der Diorganosiloxy-Einheiten in der Gruppe $Z^1$.

$V^{2*}$ = ein zweiwertiger geradkettiger, cyclischer oder verzweigter, gesättigter, ungesättigter $C_3$ bis $C_{16}$ Kohlenwasserstoffrest oder aromatischer $C_8$ bis $C_{20}$ Kohlenwasserstoffrest, der gegebenenfalls eine oder mehrere Gruppen, ausgewählt aus -O-, -CONH-, -CONR$^2$-, -C(O)-, -C(S)- enthalten kann und durch eine oder mehrere OH-Gruppe substituiert sein kann, worin

$R^2$ = Wasserstoff, ein einwertiger geradkettiger, cyclischer oder verzweigter, gesättigter, ungesättigter $C_1$ bis $C_{16}$ Kohlenwasserstoffrest oder aromatischer $C_6$ bis $C_{16}$ Kohlenwasserstoffrest ist, der eine oder mehrere Gruppen ausgewählt aus -O-, - NH-, -C(O)-, -C(S)- enthalten kann, und der gegebenenfalls durch eine oder mehrere Hydroxylgruppe substituiert sein kann, wobei wenn mehrere Gruppen -NR$^2$ vorliegen, diese gleich oder verschieden sein können,

Q=

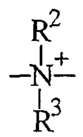

eine quaternierte Imidazoleinheit der Struktur

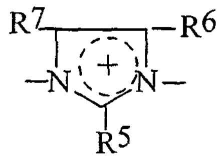

,

eine zweifach quaternierte Piperazineinheit der Struktur

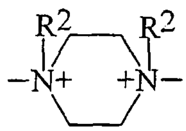

,

eine monoquaternierte Piperazineinheit der Struktur

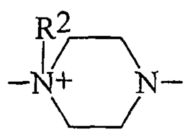

,

eine monoquaternierte Piperazineinheit der Struktur

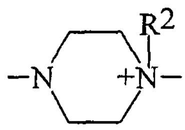

,

eine monoquaternierte Einheit der Struktur

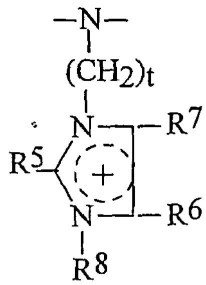

,

worin $R^2$, $R^3$, $R^4$, $R^5$, $R^6$, $R^7$ und $R^8$ wie oben definiert sind.

Besonders bevorzugt steht $V^{2*}$ für einen zweiwertigen geradkettigen, cyclischen oder verzweigten, gesättigten, ungesättigten oder aromatischen Kohlenwasserstoffrest mit bis zu 16 Kohlenstoffatomen, der eine oder mehrere Gruppen, ausgewählt aus -O-, -CONH-, $-CONR^2-$, worin $R^2$ wie oben definiert ist, -C(O)-, -C(S)- enthalten kann und mit einer oder mehreren Hydroxylgruppen substituiert sein kann. Noch bevorzugter wird $-V^{2*}-$ ausgewählt aus Gruppen der Formeln:

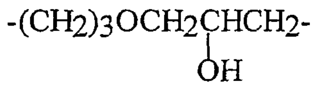

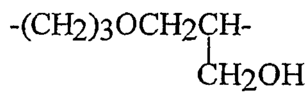

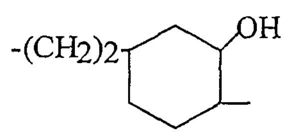

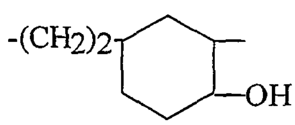

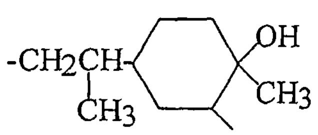

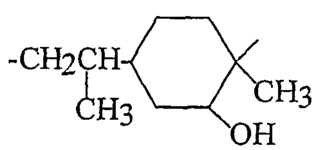

$-(CH_2)_2-$, $-(CH_2)_3-$, $-(CH_2)_4-$, $-(CH_2)_5-$, $-(CH_2)_6-$,

$-CH{=}CHCH_2-$, $-CH{=}CHCH_2CH_2-$,

$-CH_2CH_2CH_2OC(O)CH_2-$, $-CH_2CH_2CH_2OC(O)CH_2CH_2-$,

$-CH=CHCH_2OC(O)CH_2-$, $-CH=CHCH_2OC(O)CH_2CH_2-$,

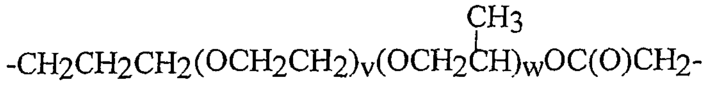

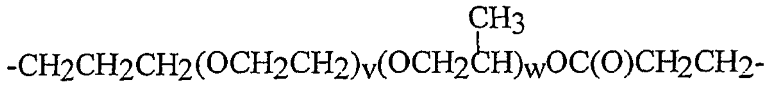

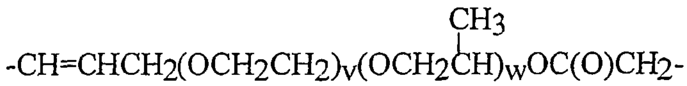

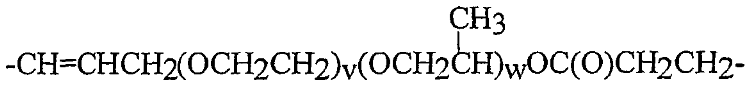

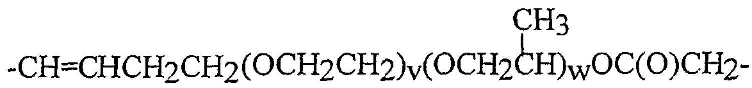

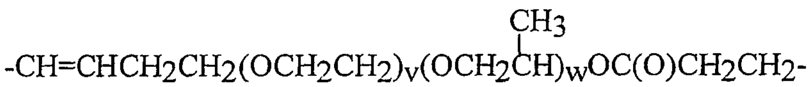

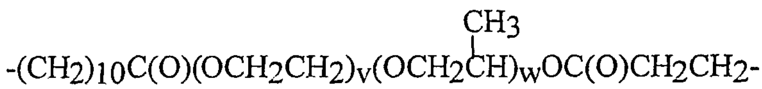

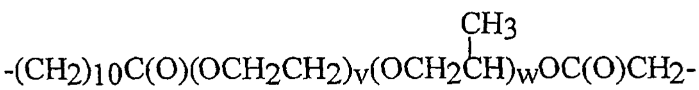

mit $v+w \geq 0$,

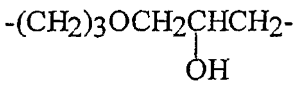

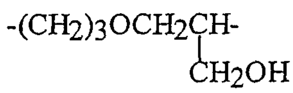

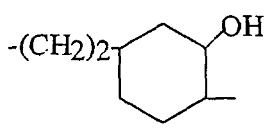

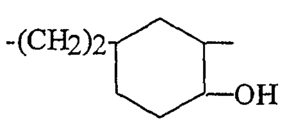

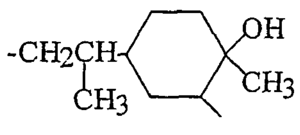

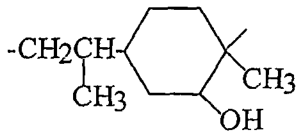

$-(CH_2)_3-$ , $-(CH_2)_4-$, $-(CH_2)_5-$, $-(CH_2)_6-$,

$-CH=CHCH_2-$, $-CH=CHCH_2CH_2-$,

$-CH_2CH_2CH_2OC(O)CH_2-$, $-CH_2CH_2CH_2OC(O)CH_2CH_2-$,

$R^2$ steht bevorzugt für:
Wasserstoff, $-CH_3$, $-CH_2CH_3$, $-(CH_2)_2CH_3$, $-(CH_2)_3CH_3$, $-(CH_2)_5CH_3$, $-CH_2CH_2OH$,

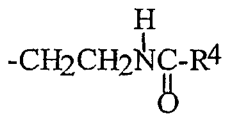

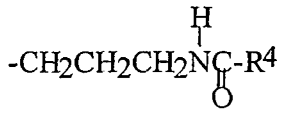

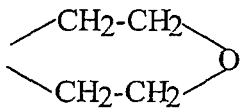

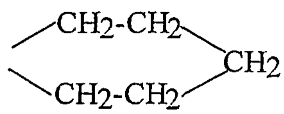

mit
$R^4$ = geradkettiger, cyclischer oder verzweigter $C_1$ bis $C_{18}$ Kohlenwasserstoffrest, der durch eine oder mehrere Gruppen, ausgewählt aus -O-, -NH-, -C(O)-, und -C(S)-enthalten kann und durch eine oder mehrere OH-Gruppen substituiert sein kann, speziell unsubstituierte $C_5$ bis $C_{17}$ Kohlenwasserstoffreste, die sich von den entsprechenden Fettäuren ableiten oder aber hydroxylierte $C_3$ bis $C_{17}$ Reste, die auf hydroxylierte Carbonsäuren, speziell Saccharidcarbonsäuren zurückgeführt werden können und ganz speziell

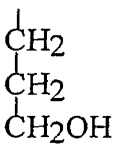

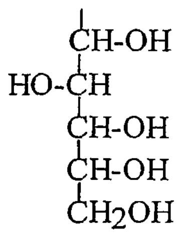

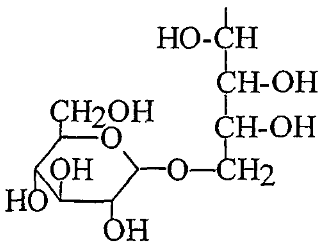

bedeuten.

Weiterhin steht $R^2$ bevorzugt für:

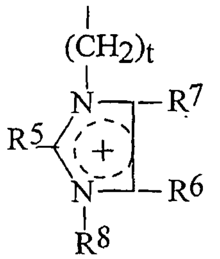

,

worin t, $R^5$ bis $R^8$ wie oben definiert sind,

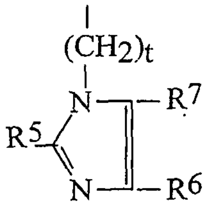

,

worin t, $R^5$ bis $R^7$ wie oben definiert sind,

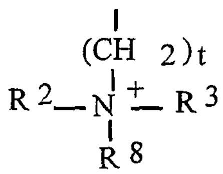

,

worin t, $R^2$, $R^3$ und $R^8$ wie oben definiert sind. $V^1$ steht bevorzugt für

- $-R^9-$, worin $R^9$ einen zweiwertigen, gesättigten oder einfach oder mehrfach ungesättigten, geradkettigen oder verzweigten Kohlenwasserstoffrest mit zwei bis 25 Kohlenstoffatomen darstellt,
- $-(CH_2)_u-R^{10}-(CH_2)_u-$, worin $R^{10}$ eine aromatische Gruppe ist,
- $-[CH_2CH_2O]_q-[CH_2CH(CH_3)O]_r-CH_2CH_2-$,
- $-CH(CH_3)CH_2O[CH_2CH_2O]_q-[CH_2CH(CH_3)O]_r-CH_2CH(CH_3)-$
- $-CH_2CH(OH)CH_2-$,
- $-CH_2CH(OH)(CH_2)_2CH(OH)CH_2-$,
- $-CH_2CH(OH)CH_2OCH_2CH(OH)CH_2OCH_2CH(OH)CH_2-$ und
- $-CH_2CH(OH)CH_2O-[CH_2CH_2O]_q-[CH_2CH(CH_3)O]_r-CH_2CH(OH)CH_2-$

worin
u von 1 bis 3 ist,
q und r von 0 bis 200, bevorzugt von 0 bis 100, bevorzugter von 0 bis 70 und besonders bevorzugt 0 bis 40 ist, und
$q + r > 0$ ist.

Bevorzugte Varianten von $V^1$ sind

Alkylen-, Alkenylen-, Alkinylen- und Aryleinheiten, speziell der Strukturen $-[CH_2]_o-$ mit o = 2 bis 6,

$-CH_2C{\equiv}CCH_2-$, $-CH_2CH{=}CHCH_2-$, $-CH(CH_3)CH_2CH_2-$,

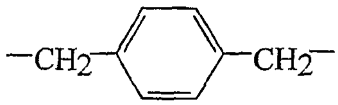

Polyalkylenoxideinheiten, speziell der Strukturen

$-[CH_2CH_2O]_q-[CH_2CH(CH_3)O]_r-CH_2CH_2-$,

$-CH(CH_3)CH_2O[CH_2CH_2O]_q-[CH_2CH(CH_3)O]_r-CH_2CH(CH_3)-$

mit
mono-, di- oder polyhydroxyfunktionelle Einheiten, speziell der Strukturen

$-CH_2CH(OH)CH_2-$, $-CH_2CH(OH)(CH_2)_2CH(OH)CH_2-$,

$-CH_2CH(OH)CH_2OCH_2CH(OH)CH_2OCH_2CH(OH)CH_2-$,

$-CH_2CH(OH)CH_2O-[CH_2CH_2O]_q-[CH_2CH(CH_3)O]_r-CH_2CH(OH)CH_2-$

mit
q = 0 bis 200,
r = 0 bis 200

Bevorzugt sind q = 1 bis 50, insbesondere 2 bis 50, speziell 1 bis 20, ganz speziell 1 bis 10, sowie 1 oder 2, r = 0 bis 100, insbesondere 0 bis 50, speziell 0 bis 20, ganz speziell 0 bis 10, sowie 0 oder 1 oder 2.

Die Erfindung betrifft weiterhin ein Verfahren zur Herstellung der erfindungsgemäßen linearen Polysiloxane, worin

a) mindestens eine Aminverbindung, ausgewählt aus einer Diamin-Verbindung und/oder einer primären oder sekundären Monoaminverbindung, mit mindestens zwei difunktionellen, zur Reaktion mit den Aminofunktionen der Amin-Verbindung befähigten organischen Verbindungen umgesetzt werden, wobei das molare Verhältnis der organischen Verbindungen so gewählt wird, dass die Bedingung $V^2/V^1 \neq 1$ erfüllt wird,

b) mindestens zwei Mol einer Aminverbindung, ausgewählt aus einer Diamin-Verbindung und/oder einer primären oder sekundären Monoaminverbindung, mit einem Mol einer difunktionellen, zur Reaktion mit den Aminofunktionen der Aminverbindung befähigten organischen Verbindung unter Bildung einer Diaminverbindung (Monomer) umgesetzt wird, die anschließend mit mindestens einer Aminverbindung, ausgewählt aus einer Diamin-Verbindung und/oder einer primären oder sekundären Monoaminverbindung, und mindestens einer weiteren difunktionellen zur Reaktion mit den Aminofunktionen der Aminverbindungen befähigten organischen Verbindung umgesetzt wird,

c) eine Aminverbindung, ausgewählt aus einer Diamin-Verbindung und/oder einer primären oder sekundären Monoaminverbindung, mit einer difunktionellen, zur Reaktion mit den Aminofunktionen der Aminverbindungen befähigten organischen Verbindung unter Bildung einer Diaminverbindung (aminoterminiertes Oligomer) umgesetzt wird, die anschließend mit mindestens einer difunktionellen zur Reaktion mit den Aminofunktionen der Diamin-Verbindungen befähigten organischen Verbindung umgesetzt wird,

d) eine Aminverbindung, ausgewählt aus einer Diamin-Verbindung und/oder einer primären oder sekundären Monoaminverbindung, mit einer difunktionellen, zur Reaktion mit den Aminofunktionen der Aminverbindung befähigten organischen Verbindung unter Bildung einer difunktionellen, zur Reaktion mit Aminofunktionen befähigten Verbindung (difunktionelles Oligomer) umgesetzt wird, die anschließend mit mindestens einer Aminverbindung, ausgewählt aus einer Diamin-Verbindung und/oder einer primären oder sekundären Monoaminverbindung, und mindestens einer weiteren zur Reaktion mit Aminofunktionen befähigten Verbindung umgesetzt wird,

wobei gegebenenfalls monofunktionelle, bevorzugt tertiäre Monoamine oder geeignete, zur Kettenfortpflanzung nicht

befähigte Monoamine und/oder monofunktionelle, zur Reaktion mit Aminofunktionen befähigten Verbindungen als Kettenabbruchsmittel hinzugesetzt werden können, und die Stöchiometrie der Aminofunktionen und der zur Reaktion mit Aminofunktionen befähigten funktionellen Gruppen in der letzten Stufe der Umsetzung stets etwa 1:1 beträgt,
und wobei gegebenenfalls vorhandene Aminofunktionen protoniert, alkyliert oder quaterniert werden können.

Variante a), worin mindestens eine Diamin-Verbindung, ausgewählt aus einer Diamin-Verbindung und/oder einer primären oder sekundären Monoaminverbindung, mit mindestens zwei difunktionellen, zur Reaktion mit den Aminofunktionen der Aminverbindung befähigten organischen Verbindungen umgesetzt werden, wobei das molare Verhältnis der organischen Verbindungen so gewählt wird, dass die Bedingung $V^2/V^1 \neq 1$ erfüllt wird, lässt sich somit schematisch beispielsweise wie folgt darstellen:

-[N-N]- + -[$V^1$]- + -[$V^2$]- → -[Q-($V^1$,$V^2$)]- oder
-[N]- + -[$V^1$]- + -[$V^2$]- → -[Q-($V^1$,$V^2$)]-

wobei -[N-N]- ein cyclisches, der Definition von Q entsprechendes Diamin oder ein $V^1$-enthaltendes Diamin -[N-$V^1$-N]- oder ein $V^2$-enthaltendes Diamin -[N-$V^2$-N]-, wie insbesondere -[N-$V^{2*}$-$Z^2$-$V^{2*}$-N]- einschließen kann, wobei aus den letzteren jeweils zwei Q-Einheiten und eine $V^1$ bzw. zwei $V^2$-Einheiten hervorgehen, und -[$V^1$]- und -[$V^2$]- den Wiederholungseinheiten $V^1$ und $V^2$ entsprechende Monomere darstellen sollen,
und -[N]- ein primäres oder sekundäres zur Kettenfortpflanzung geeignetes Monoamin darstellt,

Aus den -[N-N]- und/oder -[N]-Einheiten wird dabei mindestens eine höheralkylierte Amin- oder eine quaternäre Ammonium-Einheit Q gebildet, wobei bei der Polymerisation gebildete sekundäre oder tertiäre Aminofunktionen gegebenenfalls nach der Polymerisation in einem separaten Schritt protoniert oder quaterniert werden können. Bevorzugt ist die Bildung quarternärer Ammoniumeinheiten.

Bevorzugte Beispiele von -[N-N]- sind wie unten noch ausführlicher beschrieben wird: Piperazin und Imidazol, bevorzugte Diamin-Einheiten- [N-$V^1$-N]- schließen beispielsweise ein: Polymethylendiamine, wie Tetramethyl-Hexamethylendiamin, α,ω-diaminoterminierte Polyether, wie z.B. Jeffamine, etc.

Bevorzugte Diamin-Einheiten -[N-$V^{2*}$-$Z^2$-$V^{2*}$-N]- schließen beispielsweise Umsetzungprodukte von α,ω-Dihydrogenpolydialkylsiloxane mit Allylaminen ein. Bevorzugte Beispiele von -[N]- sind wie unten noch ausführlicher beschrieben z.B. Dimethylamin.
Die Verwendung von Diaminen -[N-N]- ist an sich bevorzugt.
Bevorzugte -[$V^1$]-Monomere schließen beispielweise Epichlorhydrin, Bisepoxide oder Bisacrylate. Es können bevorzugt auch Mischungen der genannten -[$V^1$]-Monomere, wie z.B. Mischungen aus Epichlorhydrin, Bis-Chloralkylester oder Bisepoxiden umgesetzt werden.
Bevorzugte -[$V^2$]-Monomere sind Monomere der Formel -[$V^{2*}$-$Z^2$-$V^{2*}$]-, worin $Z^2$ wie oben definiert ist, und -[$V^{2*}$] eine funktionalisierte der Wiederholungseinheit $V^{2*}$ entsprechende Gruppe darstellt. Bevorzugte -[$V^2$]-Monomere zur Bildung der $V^2$-Wiederholungseinheiten sind insbesondere α,ω-diepoxyterminierte Polydialkylsiloxane.

Variante b) lässt sich sowohl mit Diaminen, -[N-N]-, als auch geeigneten Monoaminen -[N]- durchführen und lässt sich schematisch beispielsweise wie folgt darstellen:

Variante b1)
Schritt 1): 2 -[N-N]- + -[$V^2$]- oder -[$V^1$]- → -[N-N-$V^1$-N-N]- oder -[N-N-$V^2$-N-N]-
Schritt 2.1): -[N-N-$V^2$-N-N]- + -[$V^1$]- + -[N-N]- →,
Schritt 2.2): -[N-N-$V^1$-N-N]-+-[$V^2$]-+-[N-N]- →,
wobei die Stöchiometrie so gewählt wird, dass die Bedingung $V^2/V^1 < 1 : 3$ erfüllt ist.
Bezüglich der bevorzugt verwendeten Monomer-Einheiten -[N-N]-, -[$V^1$]- und -[$V^2$]-gilt das für Schritt a) Gesagte.

Variante b2)
Schritt 1): 2 -[N]- + -[$V^2$]- oder -[$V^1$]- → -[N-V'-N]- oder -[N-$V^2$-N]-
Schritt 2.1): -[N-$V^2$-N]- +-[$V^1$]- + -[N]- →,
Schritt 2.2): -[N-$V^1$-N]- +-[$V^2$]- +-[N]- →,
wobei diese Variante wie oben erwähnt nur mit primären oder sekundären Monoaminen durchführbar ist und wobei bezüglich der bevorzugt verwendeten Monomer-Einheiten -[N]-, -[$V^1$]- und -[$V^2$]- das für Schritt a) Gesagte gilt.

Variante c) lässt sich schematisch beispielsweise wie folgt darstellen:

Variante c1)
Schritt 1): -[N-N]-+ -[$V^1$]- → -[N-N-($V^1$-N-N)$_x$]-
Schritt 2): -[N-N-($V^1$-N-N)$_x$]- + -[$V^2$]- →
wobei bezüglich der bevorzugt verwendeten Monomer-Einheiten -[N-N]-, -[$V^1$]- und -[$V^2$]- das für Schritt a) Gesagte gilt.

Variante c2)
Schritt 1): -[N]-+ -[$V^1$]- → -[N-($V^1$-N)$_x$]-
Schritt 2): -[N-($V^1$-N)$_x$]- + -[$V^2$]- →
wobei bezüglich der bevorzugt verwendeten Monomer-Einheiten -[N]-, -[$V^1$]- und - [$V^2$]- das für Schritt a) Gesagte gilt.

Variante d) lässt sich schematisch beispielsweise wie folgt darstellen:

Variante d1)
Schritt 1): -[$V^1$]- +-[N-N]- → -[$V^1$-(N-N-$V^1$)$_x$]-
Schritt 2): -[$V^1$-(N-N-$V^1$)$_x$]- + -[$V^2$]- + -[N]- oder -[N-N]- →
wobei bezüglich der bevorzugt verwendeten Monomer-Einheiten -[N-N]-, -[$V^1$]- und -[$V^2$]- das für Schritt a) Gesagte gilt.

Variante d2)
Schritt 1): -[$V^1$]- +-[N]- → -[$V^1$-(N-$V^1$)$_x$]-
Schritt 2): -[$V^1$-(N-$V^1$)$_x$]- +-[$V^2$]- + -[N]- oder -[N-N]-→

wobei bezüglich der bevorzugt verwendeten Monomer-Einheiten -[N]-, -[$V^1$]- und - [$V^2$]- das für Schritt a) Gesagte gilt.

Für alle oben schematisch dargestellten Varianten gilt, dass auch Mischungen von Monoaminen -[N]- und Diaminen -[N-N]- eingesetzt werden können.

Besonders bevorzugt werden die funktionellen Gruppen der difunktionellen, zur Reaktion mit Aminofunktionen befähigten Verbindungen ausgewählt aus der Gruppe die besteht aus Epoxygruppen und Halogenalkylgruppen.

Als Ausgangspunkt für die Synthesen der erfindungsgemäßen linearen Polysiloxancopolymere sind α,ω Si-H funktionalisierte Siloxane der allgemeinen Struktur

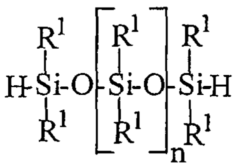

bevorzugt, wobei $R^1$ die oben angegebenen Bedeutung hat und n je nach gewünschter Wiederholungseinheit $V^1$ oder $V^2$, $n_2$ oder $n_1$ ist, die wie oben definiert sind. Sofern nicht kommerziell erhältlich, können diese Siloxane nach bekannten Verfahren, z.B. durch Äquilibrierung hergestellt werden (Silicone, Chemie und Technologie, Vulkan-Verlag, Essen 1989, S. 82-84).

Die einleitende Einführung der Strukturelemente $V^{2*}$ und Q kann z. B. auf zwei Wegen erfolgen.

Einerseits ist es möglich, zunächst tertiäre Aminofunktionen tragende ungesättigte Strukturen, beispielsweise N,N-Dimethylallylamin, durch Hydrosilylierung direkt an das Siloxan in α,ω-Stellung zu binden. Dieser Prozeß ist allgemein bekannt (B. Marciniec, Comprehensive Handbook on Hydrosilylation, Pergamon Press, Oxford 1992, S. 122-124).

Andererseits ist bevorzugt, durch Hydrosilylierung zunächst reaktive α,ω-funktionalisierte Zwischenprodukte zu erzeugen, welche nachfolgend in α,ω-ditertiäre Aminostrukturen oder direkt in die erfindungsgemäßen quartären Ammoniumstrukturen umgewandelt werden können. Geeignete Ausgangsstoffe zur Erzeugung reaktiver Zwischenstufen sind beispielsweise halogenierte Alkene oder Alkine, speziell Allylchlorid, Allylbromid, Chlorpropin und Chlorbutin, ungesättigte Halogencarbonsäureester, speziell Chloressigsäureallylester, Chloressigsäurepropargylester, 3-Chlorpropionsäureallylester und 3-Chlorpropionsäurepropargylester und epoxyfunktionelle Alkene, beispielsweise Vinylcyclohexenoxid und Allylglycidether. Die allgemeine Durchführung von Hydrosilylierungen mit Vertretern der genannten Stoffgruppen ist ebenfalls bekannt (B. Marciniec, Comprehensive Handbook on Hydrosilylation, Pergamon Press, Oxford 1992, S. 116-121, 127-130, 134-137, 151-155).
In einem nachfolgenden Schritt können die reaktiven Zwischenstufen dann mit sekundäre Aminofunktionen tragenden Verbindungen zur Reaktion gebracht werden. Geeignete Vertreter sind N,N-Dialkylamine, beispielsweise Dimethylamin, Diethylamin, Dibutylamin, Diethanolamin und N-Methylglucamin, cyclische sekundäre Amine, beispielsweise Morpholin und Piperidin, sekundäre Aminofunktionen tragende Aminoamide, beispielsweise die Umsetzungsprodukte von Diethylentriamin oder Dipropylentriamin mit Lactonen, wie γ-Butyrolacton, Gluconsäure-δ-lacton und Glucopyranosylarabonsäurelacton (DE-OS 4318536, Beispiele 11 a, 12a, 13a), oder sekundär-tertiäre Diamine, wie beispielsweise N-Methylpiperazin. Es ist speziell bevorzugt, entsprechende Imidazol- oder Pyrazolderivate, speziell Imidazol und Pyrazol zur Einführung tertiärer Aminofunktionen zu nutzen.
Als Partner für die in einer Ausführungsform bevorzugt eingesetzten Epoxidderivate eignen sich besonders die genannten sekundär-tertiären Diamine, sowie auch Imidazol und Pyrazol. Auf diese Weise können die Alkylierungen regioselektiv und ohne zusätzlichen Aufwand an die Wasserstoffatome tragenden Stickstoffatome dirigiert werden.
Zur Absicherung einer quantitativen Umwandlung der reaktiven Gruppierungen in tertiäre Aminostrukturen werden die Amine in einem Verhältnis von $1 \leq \Sigma$ sekundäre Aminogruppen : reaktive Gruppen $\leq 10$, bevorzugt 1 bis 3, speziell 1 bis 2, ganz speziell 1 eingesetzt. Aminüberschüsse müssen ggf. entfernt werden.
Die Anbindung der vorstehend beschriebenen $\alpha,\omega$-ditertiären Aminosiloxane an $V^1$ entsprechende Monomer-Einheiten -[$V^1$]- oder eine Präpolymereinheit -[$V^1$-(Q-$V^1$)$_x$]- führt zur Ausbildung von höher alkylierten Amin- bzw. quaternären Ammoniumeinheiten und kann wiederum auf zwei vorteilhaften Wegen erfolgen. Einerseits ist es bevorzugt, separat ein stark hydrophiles, polyquaternäres, difunktionelles Vorkondensat -[$V^1$-(Q-$V^1$)$_x$]- zu erzeugen, welches zu einem geeigneten Zeitpunkt mit den $\alpha,\omega$-ditertiären Aminosiloxanen vereinigt wird und zum polyquaternären Siloxancopolymeren reagiert.

Die Herstellung hoch geladener, difunktioneller Präpolymere unterschiedlicher Kettenlänge -[$V^1$-(Q-$V^1$)$_x$]- ist beispielhaft in WO 99/14300 (Beispiele 1 bis 7, Tabelle 11) beschrieben. In Abhängigkeit vom molaren Verhältnis von $V^1$ und dem Q zugrunde liegenden Amin kann entweder ein durch Aminogruppen terminiertes oder ein durch andere Reaktivgruppen (z.B. Epoxy- bzw. Halogenalkylgruppen) terminiertes Präpolymer erzeugt werden.

Für den Fall der Anbindung eines durch Aminogruppen terminierten Präpolymer -[N-($V^1$-N)$_x$]- an die Aminfunktion einer $\alpha,\omega$-ditertiären Aminosiloxanstruktur kann beispielsweise ein der Wiederholungseinheit $V^1$ entsprechendes, alkylierendes bzw. quaternierendes, difunktionelles Monomer -[$V^1$]-, ausgewählt beispielsweise aus Bisepoxiden, Epichlorhydin, Bishalogenalkyl-Verbindungen, verwendet werden. Es braucht dabei nicht erwähnt zu werden, das unterschiedliche Gruppen $V^1$ im Präpolymer und im Verbindungsglied zwischen Präpolymer und $\alpha,\omega$-ditertiärer Aminosiloxanstruktur resultieren können.

Für den Fall eines durch Reaktivgruppen terminierten Präpolymers, wie -[$V^1$-(Q-$V^1$)$_x$]- kann eine direkte Anbindung an die Aminfunktion der $\alpha,\omega$-ditertiären Aminosiloxanstruktur ohne weiteren Linker erfolgen, da bei der Präpolymersynthese bereits ein Überschuß der $V^1$ erzeugenden Komponente eingesetzt wurde.

Alternativ zur separaten Herstellung eines Vorkondensates -[$V^1$-(Q-$V^1$)$_x$]- kann der Aufbau hoch geladener Blöcke parallel zum Einbau in das Copolymere erfolgen. Dies bedeutet, daß das $\alpha,\omega$-ditertiäre Aminosiloxan mit den Startkomponenten zum Aufbau von -[$V^1$-(Q-$V^1$)$_x$]-, d.h. beispielsweise -[$V^1$]- und Mono oder Diamine der oben erwähnten Bedeutung -[N]- und/oder -[N-N-]- gemeinsam vorgelegt und zur Reaktion gebracht wird.

Schließlich ist es möglich, das $\alpha,\omega$-ditertiäre Aminosiloxan mit langkettiger Siloxaneinheit $Z^2$ oder kurzkettiger Siloxaneinheit $Z^1$ bzw. das $\alpha,\omega$-difunktionelle Siloxan -[$V^{2*}$-$Z^2$-$V^{2*}$]- oder -[$V^1$]- in die vorgelegten Komponenten zum Aufbau von -[$V^1$-(Q-$V^1$)$_x$]- über einen Zeitraum schrittweise zu dosieren oder aber umgekehrt diese Komponenten dem $\alpha,\omega$-ditertiären Aminosiloxan bzw. $\alpha,\omega$-difunktionellen Siloxan schrittweise hinzuzufügen.

Eine vorgelagerte Bereitstellung von durch Aminogruppen terminierten Präpolymeren, wie z.B. -[N-($V^1$-N)$_x$]- eröffnet die Möglichkeit, direkt mit geeigneten reaktiven Zwischenstufen, beispielsweise Epoxyderivaten, die Copolymerenbildung auszuführen.

Es ist ebenfalls bevorzugt, die reaktiven Zwischenstufen und die Startkomponenten für den Aufbau von -[$V^1$-(Q-$V^1$)$_x$]- gemeinsam vorzulegen und anschließend zur Reaktion zu bringen.

Schließlich ist möglich, die reaktiven Zwischenstufen in die vorgelegten Komponenten zum Aufbau von -[$V^1$-(Q-$V^1$)$_x$]- über einen Zeitraum schrittweise zu dosieren oder aber umgekehrt diese Komponenten der reaktiven Zwischenstufe schrittweise hinzuzufügen.

Unabhängig von der Wahl eines der vorstehend beschriebenen Reaktionswege und der damit eng verbundenen Frage, ob Aminoeinheiten zunächst das Siloxan oder aber das Präpolymer terminieren, wird die Gesamtstöchiometrie so gewählt, dass die Summe der Aminofunktionen und der mit ihnen reaktionsfähigen Gruppen etwa 1:1 beträgt.

Im Rahmen der Erfindung ist es möglich, von dieser bevorzugten Gesamtstöchiometrie abzuweichen. Es werden dann allerdings Produkte erhalten, die nicht mehr die anvisierte Länge des hoch geladenen, hydrophilen Blocks -[$V^1$-(Q-$V^1$)$_x$]- aufweisen und zusätzlich einen Überschuß einer nicht abreagierten Startkomponenten hinterlassen.

Neben der vorstehend behandelten Gesamtstöchiometrie der Reaktion ist für das Eigenschaftsbild der Produkte die Wahl der die Wiederholungseinheit $V^1$ bildenden Komponente(n) von großer Bedeutung.

Die Einführung von Alkylen-, Alkenylen-, Alkinylen- und Aryleinheiten erfolgt vorzugsweise ausgehend von den entsprechenden Halogeniden, speziell Chloriden und Bromiden. Beispielhafte Vertreter sind 1,6-Dichlorhexan, 1,4-Dichlorbut(2-)en, 1, 4-Dichlorbut(2-)in und 1,4-Bis(chlormethyl)benzol.

Polyalkylenoxideinheiten können ebenfalls über die $\alpha,\omega$-Dihalogenverbindungen eingeführt werden. Diese sind aus den oligomeren und polymeren Alkylenoxiden der allgemeinen Zusammensetzung

$$HO[CH_2CH_2O]_q-[CH_2CH(CH_3)O]_rH$$

wobei q und r die oben angegebenen Bedeutungen aufweisen, beispielsweise durch Chlorierung der Hydroxylgruppen mit $SOCl_2$ zugänglich (Organikum, Organischchemisches Grundpraktikum, 17. Auflage, VEB Deutscher Verlag der Wissenschaften, Berlin 1988, S. 189-190).

Mono-, di- oder polyhydroxyfunktionelle Einheiten als Gruppe $V^1$ können ausgehend von Epoxidderivaten eingeführt werden.

Kommerzielle Beispiele sind 1-Chlor-2,3-Epoxypropan, der Glycerol-1,3-bis-glycidylether und Diethylenglycoldiglycidylether und Neopentylglycoldiglycidylether.

Soweit nicht kommerziell verfügbar, können die gewünschten Diepoxide beispielsweise durch Reaktion der entsprechenden Diole mit 1-Chlor-2,3-Epoxypropan unter alkalischen Bedingungen synthetisiert werden.

Es liegt im Rahmen der Erfindung, in die Struktur von $V^1$ Siloxanketten $Z^1$ einzuführen. Hieraus ergibt sich u.a. die Möglichkeit, verschieden lange Siloxanketten für den Aufbau des Gesamtmoleküls zu verwenden. Es ist eine bevorzugte Variante, in $V^1$ Siloxanketten $Z^1$ des Kettenlängenbereichs $n_2$ = 0 bis 19, bevorzugt 0 bis 15, besonders bevorzugt 0 bis 10, speziell 0 bis 5, spezieller 0, einzubauen. Geeignete Startmaterialien zum Einbau sind z.B. die entsprechenden $\alpha,\omega$-Diepoxide.

Bei der Umsetzung von Epoxiden mit primären oder sekundären Aminen ist darauf zu achten, daß für Alkylierungen von tertiären Aminogruppen ein mol H+ pro mol Epoxid/ tertiäres Amin zuzusetzen wird.

Die Wahl geeigneter Amine als Ausgangskomponenten für die Bildung von Q in der Wiederholungseinheit $-[V^1-(Q-V^1)_x]-$ bestimmt ebenfalls in hohem Maße die Molekülstruktur. Die Verwendung ditertiärer Amine (entsprechend -[N-N]-), beispielsweise N,N,N',N'-Tetramethylethylendiamin, N,N,N',N'-Tetramethyltetramethylendiamin, N,N,N',N'-Tetramethylhexamethylendiamin, N,N'-Dimethylpiperazin, führt zu Produkten, in denen jedes Stickstoffatom der Wiederholungseinheit quaterniert ist.

Die Verwendung von sekundär-tertiären Diaminen, beispielsweise N-Methylpiperazin, öffnet den Weg zu Wiederholungseinheiten $-[V^1-(Q-V^1)_x]-$, in denen tertiäre und quartäre Amin- bzw. Ammoniumstrukturen im Verhältnis 1 : 1 vorliegen. Eine teilweise oder vollständige nachträgliche Quaternierung verbliebener tertiärer Aminostrukturen stellt eine bevorzugte Variante zur Einstellung einer gewünschten hohen Dichte der quartären Ammoniumgruppen dar. Die entsprechenden aromatischen Amine Imidazol bzw. Pyrazol führen zu Produkten mit einer delokalisierten Ladung.

Bei Einsatz von primär-tertiären Diaminen, beispielsweise N,N-Dimethylpropylendiamin und 1-(3-Aminopropyl) imidazol, speziell in Kombination mit Diepoxiden, können kammartige Strukturen aufgebaut werden, für die der Quaternierungsgrad während einer abschließenden Alkylierung wählbar ist. Grundsätzlich können die Alkylierungen auch zu Quaternierungsgraden von durchschnittlich weniger als einer quartären Ammoniumgruppe pro Wiederholungseinheit $-[V^1-(Q-V^1)_x]-$ eingestellt werden. Es ist jedoch bevorzugt, mindestens ein Stickstoffatom pro Wiederholungseinheit zu quaternieren.

Ausgehend von disekundären Aminen, beispielsweise Piperazin, N,N'-Bis(2-hydroxyethyl)-hexamethylendiamin, N,N'-Bis(2-hydroxypropyl)hexamethylendiamin, können grundsätzlich auch Wiederholungseinheiten $-[V^1-(Q-V^1)_x]-$ mit einem durchschnittlichen Gehalt von weniger als einer quartären Ammoniumgruppe synthetisiert werden. Die disekundären Amine liefern hierbei zunächst polytertiär aminomodifizierte Siloxancopolymere oder aber Präpolymere, die in einer abschließenden Reaktion teilweise oder vollständig zu $-[V^1-(Q-V^1)_x]-$ quaterniert werden können. Es ist aber auch in dieser Variante bevorzugt, wenigstens ein Stickstoffatom pro Wiederholungseinheit zu quaternieren.

Als geeignete Quaternierungsagenzien kommen die allgemein bekannten Stoffgruppen wie Alkylhalogenide, Halogencarbonsäureester, Epoxidderivaten in Gegenwart von $H^+$ und Dialkylsulfate, speziell Dimethylsulfat, in Betracht.

Die Herstellung nicht kommerziell verfügbarer disekundärer Amine erfolgt in einer bevorzugten Ausführungsform ausgehend von den entsprechenden diprimären Aminen, beispielsweise Hexamethylendiamin durch Alkylierung mit Epoxiden, wie z.B. Ethylenoxid, Propylenoxid, Isopropylglycidether unter Ausnutzung der unterschiedlichen Reaktionsgeschwindigkeiten primärer und sekundärer Amine.

Es war bereits dargelegt worden, daß im Rahmen der Erfindung die Möglichkeit besteht, Siloxanketten $Z^1$ in die Struktur von $V^1$ einzuführen. Als geeignete Startmaterialien wurden exemplarisch die reaktiven Zwischenstufen $\alpha,\omega$-Diepoxide benannt.

Als die aus den Ammoniumgruppen resultierenden positiven Ladungen neutralisierende Anionen $A^-$ kommen bevorzugt die während der Quaternierung gebildeten Ionen, wie Halogenidionen, speziell Chlorid und Bromid, Alkylsulfate, speziell Methosulfat, Carboxylate, speziell Acetat, Propionat, Octanoat, Decanoat, Dodecanoat, Tetradecanoat, Hexadecanoat, Octadecanoat, Oleat, Sufonate, speziell Toluensulfonat in Betracht. Jedoch können durch Ionenaustausch auch andere Anionen eingeführt werden. Zu nennen sind beispielsweise organische Anionen, wie Polyethercarboxylate und Polyethersulfate.

Die Quaternierungsreaktionen werden bevorzugt in Wasser, polaren organischen Lösungsmitteln oder Mischungen beider genannter Komponenten ausgeführt. Geeignet sind z.B. Alkohole, speziell Methanol, Ethanol, i-Propanol und n-Butanol, Glycole, wie Ethylenglycol, Diethylenglycol, Triethylenglycol, die Methyl-, Ethyl- und Butylether der

genannten Glycole, 1,2-Propylenglycol und 1,3-Propylenglycol, Ketone, wie Aceton und Methylethylketon, Ester, wie Ethylacetat, Butylacetat und 2-Ethyl-hexylacetat, Ether, wie Tetrahydrofuran und Nitroverbindungen, wie Nitromethan. Die Wahl des Lösungsmittels richtet sich wesentlich nach der Löslichkeit der Reaktionspartner, der angestrebten Reaktionstemperatur und einer gegeebenfalls vorhandenen, die Umsetzung störenden Reaktivität.

Die Reaktionen werden im Bereich von 20°C bis 130°C, vorzugsweise 40°C bis 100°C ausgeführt.

Um die Bildung von gelartigen, nicht vollständig löslichen, linearen Polyorganosiloxanpolymeren zu vermeiden, wird das Molgewicht zweckmäßig nach oben begrenzt.

Eine Begrenzung des Molekulargewichtes wird durch die sich bei der Reaktion zwischen Epoxiden, und im Reaktionssystem gegebenenfalls vorhandenem Wasser bzw. Alkohol entstehende Endstoppung oder alternativ durch die zusätzliche Verwendung von tertiären Aminen, wie Trialkylaminen oder monofunktionellen gegenüber Aminogruppen reaktive Verbindungen bewirkt.

D.h., die linearen Polyorganosiloxanpolymere können neben den naturgemäß aus der Umsetzung der monomeren Ausgangsmaterialien resultierenden terminalen Gruppen auch aus monofunktionellen Kettenabbruchsmitteln, wie Trialkylaminen etc. und z.B. daraus resultierende Ammonium-, Amino-, Ether- oder Hydroxy-Endgruppen aufweisen.

Die vorliegende Erfindung betrifft weiterhin die Verwendung der erfindungsgemäßen linearen Polyorganosiloxanpolymere bzw. der nach dem Verfahren der Erfindung erhaltenen linearen Polyorganosiloxanpolymere in kosmetischen Formulierungen, in Waschmitteln oder zur Oberflächenbehandlung von Substraten.

Die erfindungsgemäßen linearen Polyorganosiloxanpolymere, welche in sich die weichmachenden Eigenschaften von Siloxanstrukturen, und die Tendenz von quartären Ammoniumgruppen zur Adsorption an negativ geladenen Festkörperoberflächen vereinen, können mit Erfolg eingesetzt werden in kosmetischen Formulierungen für die Haut- und Haarpflege, in Polituren für die Behandlung und Ausrüstung harter Oberflächen, in Formulierungen zum Trocknen von Automobilen und anderen harten Oberflächen nach maschinellen Wäschen, zur Ausrüstung von Textilen, Textilfasern, Papier, Papierfasern, Papiervliese, einschließlich Faser-, Textil- und Papiervor- und endbehandlung, Ausrüstung von Papier für den Kosmetik- und Sanitärbereich, besonders permanente hydrophile Weichmacher, als separate Weichmacher nach dem Waschen von Textilien mit anionischen/nichtionogen Detergenzienformulierungen, als Weichmacher in auf anionischen/nichtionogenen Tensiden beruhenden Formulierungen zur Textilwäsche, sowie als Bügelhilfe und als Mittel zur Verhinderung bzw. Rückgängigmachung von Textilverknitterungen. Die Erfindung betrifft weiterhin Zusammensetzungen, enthaltend mindestens eines der erfindungsgemäßen linearen Polyorganosiloxanpolymere zusammen mit mindestens einem weiteren für die Zusammensetzung üblichen Inhaltsstoff, wie kosmetische Zusammensetzungen, Waschmittelzusammensetzungen, Polituren, Shampoos, Bügelhilfen, Knitterfreiausrüstungen.

Die Verwendung der erfindungsgemäßen Polysiloxanderivate führt bei Anwendung im Haarkosmetikbereich zu günstigen Effekten hinsichtlich Glanz, Fixierung (Halt), Körper, Volumen, Feuchtigkeitsregulierung, Farbretention, Schutz vor Umwelteinflüssen (UV, Salzwasser usw.), Wiederformbarkeit, antistatischen Eigenschaften, Färbbarkeit, Kämmbarkeit etc. D.h. die quartären Polysiloxanverbindungen können bevorzugt in den Kosmetik- und Haarpflegerezepturen gemäss der WO 02-10257 eingesetzt werden.

<u>Beispiele</u>

<u>Beispiel 1</u>

19.38g (0.225mol Aminogruppen) N,N,N',N'-Tetramethylhexandiamin und 12.14g (0.202mol) Essigsäure werden mit 30 ml deionisierten Wassers bei Raumtemperatur gemischt. Zu dieser Lösung werden innerhalb 15 Minuten 35.26g (0.202mol Epoxygruppen) einer 50%igen Lösung von Ethylenglycoldiglycidylether in Ethylenglycoldimethylether getropft. Die Temperatur steigt auf 92°C an. Innerhalb einer Nachreaktionszeit von 20 Minuten entsteht eine gelartige Masse. Diese Gelmasse wird einer Mischung zugesetzt, die aus 150g (0.025mol Epoxygruppen) eines Epoxysiloxans der Struktur

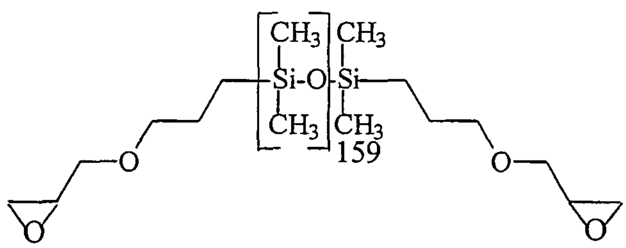

0.75g (0.0125) Essigsäure, 2.5g (0.0125mol) Dodecansäure, 0.33g (0.0025mol; 45%ige wässrige Lösung)

Trimethylamin und 50ml 2-Propanol besteht. Die Reaktion erfolgt über 16 Stunden bei 90°C. Anschließend werden bei 20hPa/80°C alle flüchtigen Bestandteile abgezogen. Es werden 182g eines weißen, festen bis wachsartigen Materials erhalten. Die folgende Formel zeigt die quantitative Zusammensetzung:

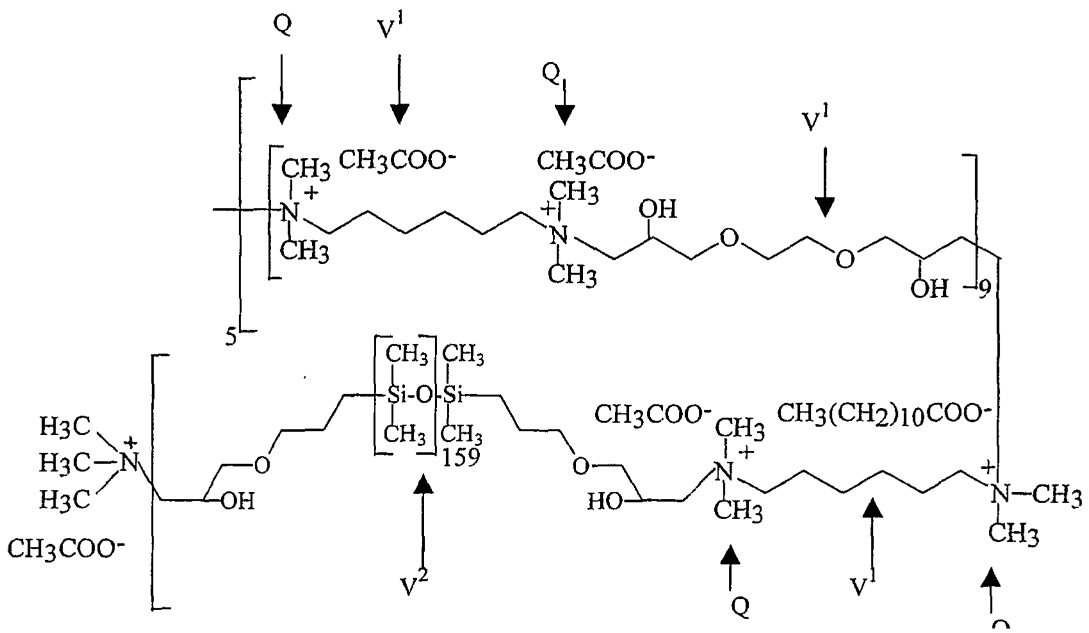

Das Verhältnis $V^2/V^1$ ist bei diesem Beispiel ca. 0,058.

Beispiel 2

27.6g (0.255mol Epoxygruppen) Neopentyldiglycidylether und 54.8g (0.0316mol Epoxygruppen) eines Siloxans der Struktur

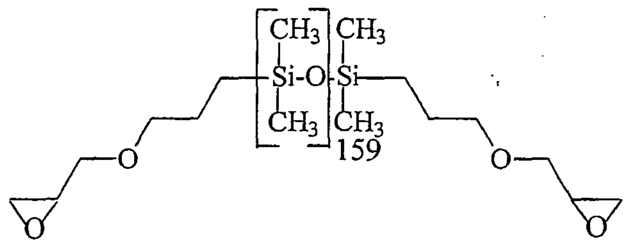

werden bei Raumtemperatur in 200 ml 2-Propanol gelöst. Zu dieser Lösung werden 17.8g (0.142mol primäre Aminogruppen) 1-(3-Aminopropyl)imidazol gegeben. Die Ringöffnungsreaktion erfolgt für 8 Stunden bei 80°C. Anschließend werden 17.9g (0.142mol) Dimethylsulfat zugesetzt und innerhalb 5 Stunden die Quaternierungsreaktion durchgeführt. Reste von Dimethylsulfat werden durch Zugabe von 10ml Wasser zersetzt. Nach Abziehen aller bis 20hPa/60°C siedenden Bestandteile werden 97.5g eines braunen, trüben Produktes erhalten. Die folgende Formel zeigt die quantitative Zusammensetzung:

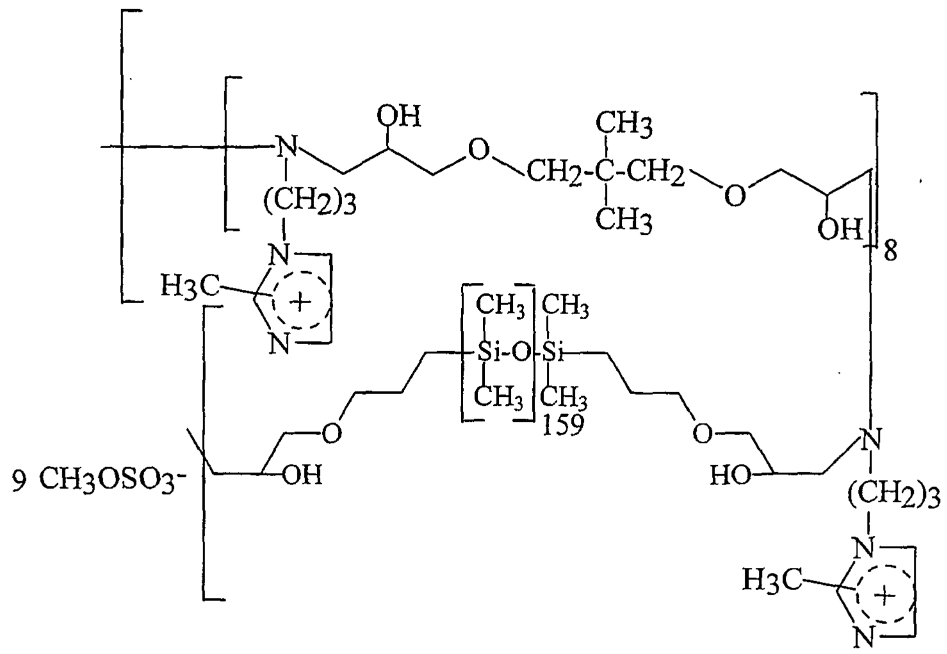

Das Verhältnis $V^2/V^1$ ist bei diesem Beispiel ca. 0,12.

<u>Beispiel 3</u>

9.67g (0.112mol Aminogruppen) N,N,N',N'-Tetramethylhexandiamin, 0,17g (0.0013mol) einer 45%igen wässrigen Trimethylaminlösung, 11,35g (0.056 mol) Dodecansäure und 3.4g (0.056mol) Essigsäure werden mit 6 ml deionisierten Wassers und 124g 2-Propanol bei Raumtemperatur gemischt und auf 50°C erhitzt. In die klare Lösung werden 86.85g (0.0124 mol Epoxygruppen) eines Epoxysiloxans der Struktur

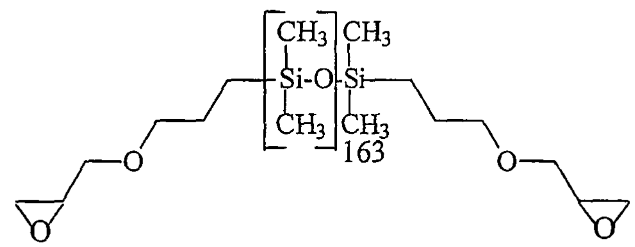

und 18.28g (0.101 mol Epoxygruppen) eines Epoxysiloxans der Struktur

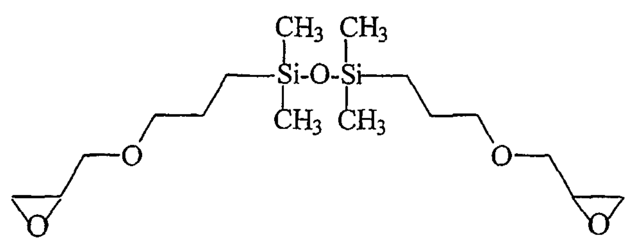

eingetropft. Der Reaktionsansatz wird auf 84°C erhitzt und diese Temperatur 14.5 Stunden aufrecht erhalten. Nach 15 Minuten wurde eine beginnende Eintrübung beobachtet. Nach Abschluß der Reaktion wird der Ansatz geteilt. Aus der

einen Hälfte des Ansatzes werden bei 20hPa/80°C alle flüchtigen Bestandteile abgezogen. Es werden 54g einer zähviskosen, fast weißen Masse erhalten. Aus der anderen Ansatzhälfte werden die flüchigen Bestandteile bei 20hPa/25°C entfernt. Es werden 58g eines leicht gelblichen, viskosen Öls gewonnen. Die folgende Formel zeigt die quantitative Zusammensetzung:

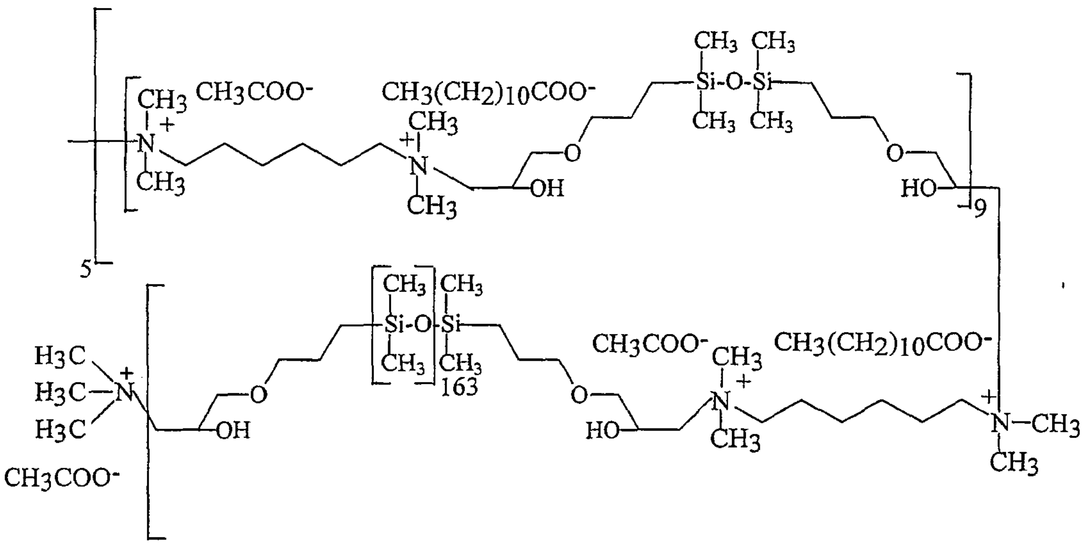

Das Verhältnis $V^2/V^1$ ist bei diesem Beispiel ca. 0,058.

## Patentansprüche

1. Lineare Polyammonium-Polysiloxancopolymere mit der Wiederholungseinheit:

-[Q-V]- (I)

worin Q aus der Gruppe ausgewählt wird, die besteht aus:

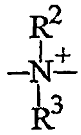

einer quaternierten Imidazoleinheit der Struktur

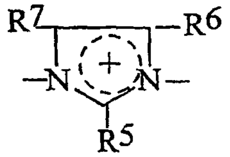

,

einer quaternierten Pyrazoleinheit der Struktur

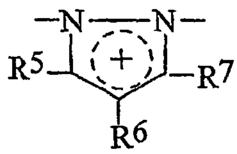

einer zweifach quaternierten Piperazineinheit der Struktur

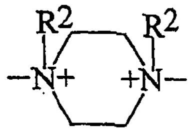

,

einer monoquaternierten Piperazineinheit der Struktur

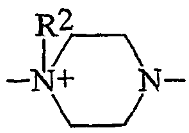

,

einer monoquaternierten Piperazineinheit der Struktur

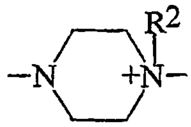

,

einer monoquaternierten Einheit der Struktur

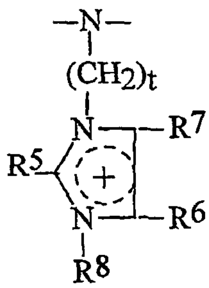

,

worin $R^2$ einen einwertigen, geradkettigen, cyclischen oder verzweigten, gesättigten, ungesättigten oder aromatischen Kohlenwasserstoffrest mit bis zu 100 Kohlenstoffatomen darstellt, der eine oder mehrere Gruppen ausgewählt aus -O-, -NH-, -C(O)- und -C(S)- enthalten kann, und der gegebenenfalls durch eine oder mehrere Substituenten, ausgewählt aus der Gruppe, die besteht aus einer Hydroxylgruppe, einer gegebenenfalls substituierten, bevorzugt ein oder mehrere Stickstoffatome enthaltenden heterocyclischen Gruppe, Amino, Alkylamino, Dialkylamino, Ammonium, Polyetherresten und Polyetheresterresten substituiert sein kann, wobei wenn mehrere Gruppen - $CONR^2$- vorliegen, diese gleich oder verschieden sein können,
$R^3$ die Bedeutung von $R^2$ aufweist, wobei $R^2$ und $R^3$ gleich oder verschieden sein können,
$R^2$ und $R^3$ gemeinsam mit dem positiv geladenen Stickstoffatom einen fünfbis siebengliedrigen Heterocyclus bilden, der gegebenenfalls zusätzlich ein oder mehrere Stickstoff-, Sauerstoff- und/oder Schwefelatome aufweisen kann,
$R^5$, $R^6$, $R^7$ gleich oder verschieden sein können und aus der Gruppe ausgewählt werden, die besteht aus: Wasserstoff, Halogen, Hydroxylgruppe, Nitrogruppe, Cyanogruppe, Thiolgruppe, Carboxylgruppe, Alkylgruppe, Monohydroxyalkylgruppe, Polyhydroxyalkylgruppe, Thioalkylgruppe, Cyanoalkylgruppe, Alkoxygruppe, Acylgruppe, Acetyloxygruppe, Cycloalkylgruppe, Arylgruppe, Alkylarylgruppe, und Gruppen des Typs -$NHR^W$, in denen $R^W$ Wasserstoff, Alkylgruppe, Monohydroxyalkylgruppe, Polyhydroxyalkylgruppe, Acetylgruppe, Ureidogruppe bedeuten, und jeweils zwei der benachbarten Reste $R^5$, $R^6$ und $R^7$ mit den sie an den Heterocyclus bindenden Kohlenstoffatomen aromatische Fünf bis Siebenringe bilden können,und
$R^8$ die Bedeutung von $R^2$ aufweist, wobei $R^8$ und $R^2$ gleich oder verschieden sein können,

wobei Q nicht an ein Carbonylkofilensioffatom bindet,
V mindestens eine Gruppe $V^1$ und mindestens eine Gruppe $V^2$ darstellt,
worin
$V^2$ ausgewählt wird aus zweiwertigen oder dreiwertigen, geradkettigen, cyclischen oder verzweigten, gesättigten, ungesättigten oder aromatischen Kohlenwasserstoffresten mit bis zu 1000 Kohlenstoffatomen (wobei die Kohlenstoffatome des unten definierten Polysiloxanrestes $Z^2$ nicht mitgezählt werden), die gegebenenfalls eine oder mehrere Gruppen, ausgewählt aus

-O-, -CONH-,

-$CONR^2$-, worin $R^2$ wie oben definiert ist,

-C(O)- und -C(S)- enthalten kann, und

der Rest $V^2$ gegebenenfalls durch eine oder mehrere Hydroxylgruppen substituiert sein kann, und
der Rest $V^2$ mindestens eine Gruppe -$Z^2$- der Formel

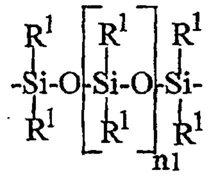

enthält, worin
$R^1$ gleich oder verschieden sein kann und aus der Gruppe ausgewählt wird, die besteht aus: $C_1$ bis $C_{22}$ Alkyl, Fluor ($C_1$-$C_{10}$)alkyl und $C_6$-$C_{10}$ Aryl, und $n_1$ = 20 bis 1000 bedeutet,
$V^1$ ausgewählt wird aus zweiwertigen oder dreiwertigen, geradkettigen, cyclischen oder verzweigten, gesättigten, ungesättigten oder aromatischen Kohlenwasserstoffresten mit bis zu 1000 Kohlenstoffatomen, die gegebenenfalls eine oder mehrere Gruppen, ausgewählt aus

-O-, -CONH-,

-$CONR^2$-, worin $R^2$ wie oben definiert ist, wobei die Gruppen $R^2$ in den Gruppen $V^1$ und $V^2$ gleich oder verschieden sein können,
-C(O)-, -C(S)- und -$Z^1$- enthalten kann, worin -$Z^1$- eine Gruppe der Formel

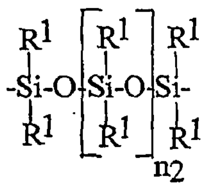

ist, worin

$R^1$ wie oben definiert ist, wobei die Gruppen $R^1$ in den Gruppen $V^1$ und $V^2$ gleich oder verschieden sein können, und
$n_2$ = 0 bis 19 bedeutet,

und der Rest $V^1$ gegebenenfalls durch eine oder mehrere Hydroxylgruppen substituiert sein kann, mit der Maßgabe,

- dass der Rest $V^1$ keine Estergruppe(n) -C(O)-O- bzw. -O-C(O)-aufweisen darf,
- dass die dreiwertigen Reste Q und die dreiwertigen Reste $V^1$ oder $V^2$ ausschließlich der Absättigung untereinander innerhalb der linearen Hauptkette der genannten Polysiloxan-Copolymere dienen, und
- dass in dem genannten Polysiloxan-Copolynier das molare Verhältnis

$$V^2/V^1 \neq 1$$

ist,

und worin die aus den Ammoniumgruppen resultierenden positiven Ladungen durch organische oder anorganische Säureanionen neutralisiert sind,
und deren Säureadditionssalze.

**2.** Lineare Polyammonium-Polysiloxancopolymere nach Anspruch 1, worin $V^2$ eine Gruppe der Formel

$$-V^{2*}-Z^2-V^{2*}-$$

ist, worin $Z^2$ wie oben definiert ist und $V^{2*}$ einen zweiwertigen geradkettigen, cyclischen oder verzweigten, gesättigten, ungesättigten oder aromatischen Kohlenwasserstoffrest mit bis zu 40 Kohlenstoffatomen darstellt, der gegebenenfalls eine oder mehrere Gruppen, ausgewählt aus -O-, -CONH-, $-CONR^2-$, worin $R^2$ wie oben definiert ist, -C(O)- und -C(S)- enthalten kann, und der Rest $V^{2*}$ gegebenenfalls durch eine oder mehrere Hydroxylgruppen substituiert sein kann.

**3.** Lineare Polyammonium-Polysiloxancopolymere nach Anspruch 1 oder 2, worin die Gruppe $V^1$ ausgewählt wird aus zweiwertigen, geradkettigen, cyclischen oder verzweigten, gesättigten, ungesättigten oder aromatischen Kohlenwasserstoffresten mit bis zu 600 Kohlenstoffatomen, die gegebenenfalls eine oder mehrere Gruppen, ausgewählt aus -O-, -CONH-, $-CONR^2-$, worin $R^2$ wie oben definiert ist, -C(O)-, -C(S)- und $-Z^1-$ enthalten kann, worin $-Z^1-$ eine Gruppe der Formel

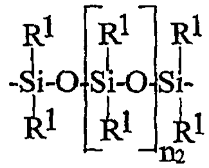

ist, worin

$R^1$ $C_1$ bis $C_3$Alkyl, Fluor($C_3$-$C_6$)alkyl oder $C_6$-Aryl ist, und

$n_2$ wie oben definiert ist.

4. Lineare Polyammonium-Polysiloxancopolymere nach einem der Ansprüche 1 bis 3, worin das molare Verhältnis $V^2/V^1$ die Beziehung

$$V^2/V^1 < 1$$

erfüllt.

5. Lineare Polyammonium-Polysiloxancopolymere nach einem der Ansprüche 1 bis 4, worin das molare Verhältnis $V^2/V^1$ die Beziehung

$$0.0005 < V^2/V^1 < 0,9$$

erfüllt.

6. Verfahren zur Herstellung der linearen Polyammonium-Polysiloxancopolymere nach einem der Ansprüche 1 bis 5, worin

a) mindestens eine Aminverbindung, ausgewählt aus einer Diamin-Verbindung und/oder einer primären oder sekundären Monoaminverbindung, mit mindestens zwei difunktionellen, zur Reaktion mit den Aminofunktionen der Amin-Verbindung befähigten organischen Verbindungen umgesetzt werden, wobei das molare Verhältnis der organischen Verbindungen so gewählt wird, dass die Bedingung $V^2/V^1 \neq 1$ erfüllt wird,
b) mindestens zwei Mol einer Aminverbindung, ausgewählt aus einer Diamin-Verbindung und/oder einer primären oder sekundären Monoaminverbindung, mit einem Mol einer difunktionellen, zur Reaktion mit den Aminofunktionen der Aminverbindung befähigten organischen Verbindung unter Bildung einer Diaminverbindung (Monomer) umgesetzt wird, die anschließend mit mindestens einer Aminverbindung, ausgewählt aus einer Diamin-Verbindung und/oder einer primären oder sekundären Monoaminverbindung, und mindestens einer weiteren difunktionellen zur Reaktion mit den Aminofunktionen der Aminverbindungen befähigten organischen Verbindung umgesetzt wird,
c) eine Aminverbindung, ausgewählt aus einer Diamin-Verbindung und/oder einer primären oder sekundären Monoaminverbindung, mit einer difunktionellen, zur Reaktion mit den Aminofunktionen der Aminverbindungen befähigten organischen Verbindung unter Bildung einer Diaminverbindung (aminoterminiertes Oligomer) umgesetzt wird, die anschließend mit mindestens einer difunktionellen zur Reaktion mit den Aminofunktionen der Diamin-Verbindungen befähigten organischen Verbindung umgesetzt wird,
d) eine Aminverbindung, ausgewählt aus einer Diamin-Verbindung und/oder einer primären oder sekundären Monoaminverbindung, mit einer difunktionellen, zur Reaktion mit den Aminofunktionen der Aminverbindung befähigten organischen Verbindung unter Bildung einer difunktionellen, zur Reaktion mit Aminofunktionen befähigten Verbindung (difunktionelles Oligomer) umgesetzt wird, die anschließend mit mindestens einer Aminverbindung, ausgewählt aus einer Diamin-Verbindung und/oder einer primären oder sekundären Monoaminverbindung, und mindestens einer weiteren zur Reaktion mit Aminofunktionen befähigten Verbindung umgesetzt wird,

wobei gegebenenfalls monofunktionelle, bevorzugt tertiäre Monoamine oder geeignete, zur Kettenfortpflanzung nicht befähigte Monoamine und/oder monofunktionelle, zur Reaktion mit Aminofunktionen befähigten Verbindungen als Kettenabbruchsmittel hinzugesetzt werden können, und die Stöchiometrie der Aminofunktionen und der zur Reaktion mit Aminofunktionen befähigten funktionellen Gruppen in der letzten Stufe der Umsetzung stets etwa 1:1 beträgt,
und wobei gegebenenfalls vorhandene Aminofunktionen protoniert, alkyliert oder quaterniert werden können.

7. Verfahren nach Anspruch 6, worin die funktionellen Gruppen der difunktionellen, zur Reaktion mit Aminofunktionen befähigten Verbindungen ausgewählt werden aus der Gruppe, die besteht aus Epoxygruppen und Halogenalkylgruppen.

8. Verwendung der linearen Polyammonium-Polysiloxancopolymere nach einem der Ansprüche 1 bis 5, sowie der linearen Polyammonium-Polysiloxancopolymere, die nach Anspruch 6 oder 7 erhalten werden, in kosmetischen Formulierungen, in Waschmitteln oder zur Oberflächenbehandlung von Substraten.

**9.** Verwendung nach Anspruch 8 zur Faserbehandlung bzw. Faserausrüstung.

**10.** Zusammensetzungen, enthaltend mindestens ein lineares Polyammonium-Polysiloxancopolymer nach irgend einem der Ansprüche 1 bis 5 oder mindestens eines der linearen Polyammonium-Polysiloxancopolymere, die nach einem der Ansprüche 6 oder 7 erhalten werden, zusammen mit mindestens einem weiteren für die Zusammensetzung üblichen Inhaltsstoff.

**11.** Zusammensetzung nach Anspruch 10, die eine Waschmittelzusammensetzung oder eine kosmetische Zusammensetzung ist.

## Claims

**1.** Linear polyammonium-polysiloxane copolymers containing the repeating unit

-[Q-V-]- (I)

in which Q is selected from the group consisting of

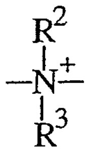

,

a quatemized imidazole unit of the structure

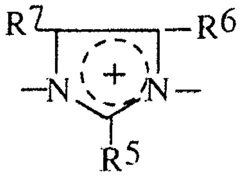

,

a quatemized pyrazole unit of the structure

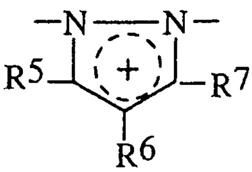

,

a diquatemized piperazine unit of the structure

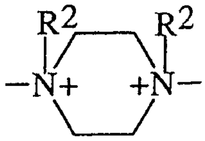

,

a monoquaternized piperazine unit of the structure

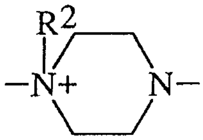

,

a monoquaternized piperazine unit of the structure

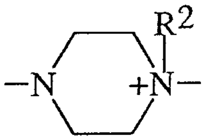

,

a monoquaternized unit of the structure

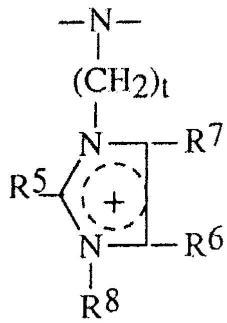

;

in which $R^2$ is a monovalent, straight-chain, cyclic or branched, saturated, unsaturated or aromatic hydrocarbon radical having up to 100 carbon atoms, which may contain one or more groups selected from -O-, -NH-, -C(O)- and -C(S)-, and which may if desired be substituted by one or more substituents selected from the group consisting of a hydroxyl group, an unsubstituted or substituted heterocyclic group preferably containing one or more nitrogen atoms, amino, alkylamino, dialkylamino, ammonium, polyether radicals and polyetherester radicals, and, if there are two or more groups $-CONR^2-$, they may be identical or different,
$R^3$ has the definition of $R^2$, it being possible for $R^2$ and $R^3$ to be identical or different, or
$R^2$ and $R^3$ together with the positively charged nitrogen atom form a five- to seven-membered heterocycle, which if desired may additionally contain one or more nitrogen, oxygen and/or sulfur atoms,
$R^5$, $R^6$ and $R^7$ can be identical or different and are selected from the group consisting of hydrogen, halogen, hydroxyl group, nitro group, cyano group, thiol group, carboxyl group, alkyl group, monohydroxyalkyl group, polyhydroxyalkyl group, thioalkyl group, cyanoalkyl group, alkoxy group, acyl group, acetyloxy group, cycloalkyl group, aryl group, alkylaryl group, and groups of the type $-NHR^W$, in which $R^W$ is hydrogen, alkyl group, monohydroxyalkyl group, polyhydroxyalkyl group, acetyl group or ureido group, and pairs of adjacent radicals $R^5$, $R^6$ and $R^7$ may, with the

carbon atoms bonding them to the heterocycle, form aromatic five- to seven-membered rings, and
$R^8$ has the definition of $R^2$, it being possible for $R^8$ and $R^2$ to be identical or different,
Q not bonding to a carbonyl carbon atom,
V represents at least one group $V^1$ and at least one group $V^2$
in which
$V^2$ is selected from divalent or trivalent, straight-chain, cyclic or branched, saturated, unsaturated or aromatic hydrocarbon radicals having up to 1000 carbon atoms (not including the carbon atoms of the polysiloxane radical $Z^2$, defined below) and containing, if desired, one or more groups selected from

-O-, -CONH-,

$-CONR^2-$, in which $R^2$ is as defined above,

-C(O)- and -C(S)-,

and
the radical $V^2$ may if desired be substituted by one or more hydroxyl groups, and
the radical $V^2$ contains at least one group $-Z^2-$ of the formula

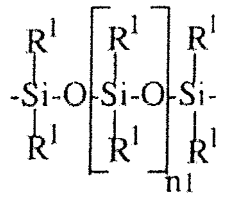

in which
$R^1$ can be identical or different and is selected from the group consisting of $C_1$ to $C_{22}$ alkyl, fluoro($C_1$-$C_{10}$)alkyl and $C_6$-$C_{10}$ aryl, and $n_1$ = 20 to 1000,
$V^1$ is selected from dihydric or trihydric, straight-chain, cyclic or branched, saturated, unsaturated or aromatic hydrocarbon radicals having up to 1000 carbon atoms, which if desired may contain one or more groups selected from

-O-, -CONH-,

$-CONR^2-$, in which $R^2$ is as defined above, it being possible for the groups $R^2$ in the groups $V^1$ and $V^2$ to be identical or different,
-C(O)-, -C(S)- and $-Z^1-$, in which $-Z^1-$ is a group of the formula

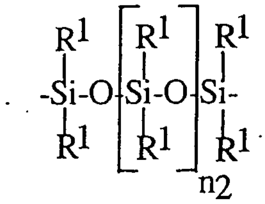

in which
$R^1$ is as defined above, it being possible for the groups $R^1$ in the groups $V^1$ and $V^2$ to be identical or different, and $n_2$ = 0 to 19,
and the radical $V^1$ may if desired be substituted by one or more hydroxyl groups,
with the provisos

- that the radical $V^1$ may not contain any ester group(s) -C(O)-O-and/or -O-C(O)-,
- that the trivalent radicals Q and the trivalent radicals $V^1$ or $V^2$ serve exclusively for saturating one another within the linear main chain of the stated polysiloxane copolymers, and

- that in the stated polysiloxane copolymer the molar ratio

$$V^2/V^1 \neq 1,$$

and in which the positive charges resulting from the ammonium groups are neutralized by organic or inorganic acid anions,
and the acid addition salts thereof.

**2.** Linear polyammonium-polysiloxane copolymers according to claim 1, in which $V^2$ is a group of the formula

$$-V^{2*}-Z^2-V^{2*}-$$

in which $Z^2$ is as defined above and $V^{2*}$ is a divalent straight-chain cyclic or branched, saturated, unsaturated or aromatic hydrocarbon radical having up to 40 carbon atoms, which if desired may contain one or more groups selected from -O-, -CONH-, $-CONR^2-$, in which $R^2$ is as defined above, -C(O)- and -C(S)-, and the radical $V^{2*}$ may if desired be substituted by one or more hydroxyl groups.

**3.** Linear polyammonium-polysiloxane copolymers according to claim 1 or 2, in which the group $V^1$ is selected from divalent, straight-chain, cyclic or branched, saturated, unsaturated or aromatic hydrocarbon radicals having up to 600 carbon atoms, which may if desired contain one or more groups selected from

- O-, -CONH-, $-CONR^2-$, in which $R^2$ is as defined above, -C(O)-, -C(S)- and $-Z^1-$, in which-$Z^1$- is a group of the formula

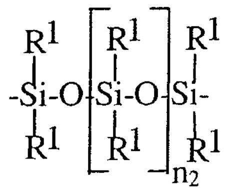

in which
$R^1$ is $C_1$ to $C_3$alkyl, fluoro($C_3$-$C_6$)alkyl or $C_6$ aryl, and
$n_2$ is as defined above.

**4.** Linear polyammonium-polysiloxane copolymers according to one of claims 1 to 3, in which the molar ratio $V^2/V^1$ complies with the relationship

$$V^2/V^1<1.$$

**5.** Linear polyammonium-polysiloxane copolymers according to one of claims 1 to 4, in which the molar ratio $V^2/V^1$ complies with the relationship

$$0.0005 < V^2/V^1 < 0.9.$$

**6.** A process for preparing the linear polyammonium-polysiloxane copolymers according to one of claims 1 to 5, in which

a) at least one amine compound selected from a diamine compound and/or a primary or secondary monoamine compound is reacted with at least two difunctional organic compounds capable of reacting with the amino functions of the amine compound, the molar ratio of the organic compounds being chosen so as to meet the condition $V^2/V^1 \neq 1$,
b) at least two moles of an amine compound selected from a diamine compound and/or a primary or secondary monoamine compound are reacted with one mole of a difunctional organic compound capable of reacting with the amino functions of the amine compound, to form a diamine compound (monomer), which is subsequently reacted with at least one amine compound selected from a diamine compound and/or a primary or secondary monoamine compound and with at least one further difunctional organic compound capable of reacting with the amino functions of the amine compounds,

c) an amine compound selected from a diamine compound and/or a primary or secondary monoamine compound is reacted with a difunctional organic compound capable of reacting with the amino functions of the amine compounds, to form a diamine compound (amino-terminated oligomer), which is subsequently reacted with at least one difunctional organic compound capable of reacting with the amino functions of the diamine compounds,
d) an amine compound selected from a diamine compound and/or a primary or secondary monoamine compound is reacted with a difunctional organic compound capable of reacting with the amino functions of the amine compound, to form a difunctional compound capable of reacting with amino functions (difunctional oligomer), which is subsequently reacted with at least one amine compound selected from a diamine compound and/or a primary or secondary monoamine compound and with at least one further compound capable of reacting with amino functions, it being possible if desired to add monofunctional, preferably tertiary, monoamines or suitable monoamines not capable of chain propagation, and/or monofunctional compounds capable of reacting with amino functions, as chain terminators, and the stoichiometry of the amino functions and the functional groups capable of reacting with amino functions always being approximately 1:1 in the last stage of the reaction,
and it being possible for any amino functions present to be protonated, alkylated or quaternized.

**7.** The process according to claim 6, in which the functional groups of the difunctional compounds capable of reacting with amino functions are selected from the group consisting of epoxy groups and haloalkyl groups.

**8.** The use of the linear polyammonium-polysiloxane copolymers according to one of claims 1 to 5 and of the linear polyammonium-polysiloxane copolymers obtained according to claim 6 or 7 in cosmetic formulations, in laundry detergents or for surface-treating substrates.

**9.** The use according to claim 8 for fiber treatment and/or fiber finishing.

**10.** Compositions comprising at least one linear polyammonium-polysiloxane copolymer according to any one of claims 1 to 5 or at least one of the linear polyammonium-polysiloxane copolymers obtained according to one of claims 6 or 7, together with at least one further ingredient customary for the composition.

**11.** A composition according to claim 10, being a laundry detergent composition or a cosmetic composition.

**Revendications**

**1.** Copolymères linéaires de polyammonium et de polysiloxane comprenant le motif répétitif :

-[Q-V] (1)

dans lequel Q est choisi dans le groupe constitué par:

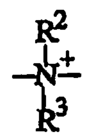

un motif imidazole quaternisé de structure

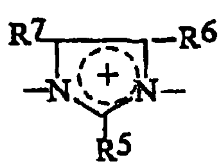

un motif pyrazole quaternisé de structure

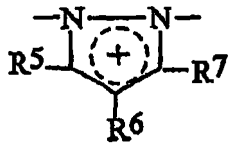

un motif pipérazine diquaternisé de structure

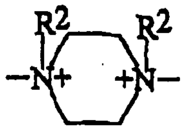

un motif pipérazine monoquaternisé de structure

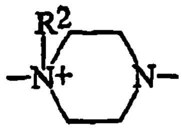

un motif pipérazine monoquaternisé de structure

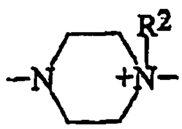

un motif monoquaternisé de structure

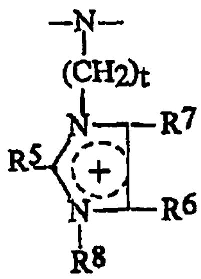

où

$R^2$ représente un reste hydrocarboné monovalent, à chaîne droite, cyclique ou ramifiée, saturé, insaturé ou aromatique comprenant jusqu'à 100 atomes de carbone, qui peut contenir un ou plusieurs groupes choisis parmi -O-, -NH-, -C(O)- et -C(S)- et qui peut être substitué le cas échéant par un ou plusieurs substituants choisis dans le groupe constitué par un groupe hydroxyle, un groupe hétérocyclique le cas échéant substitué et de préférence contenant un ou plusieurs atomes d'azote, des restes amino, alkylamino, dialkylamino, ammonio, polyéther et polyéther ester et, lorsque plusieurs groupes $-CONR^2-$ sont présents, ils peuvent être identiques ou différents,

$R^3$ a la même signification que $R^2$, $R^2$ et $R^3$ pouvant être identiques ou différents,

$R^2$ et $R^3$ pris ensemble avec l'atome d'azote chargé positivement forment un hétérocycle de cinq à sept termes qui peut le cas échéant contenir en plus un ou plusieurs atomes d'azote, d'oxygène et/ou de soufre,

$R^5$, $R^6$, $R^7$ peuvent être identiques ou différents et sont choisis dans le groupe constitué par l'hydrogène, un halogène, un groupe hydroxyle, un groupe nitro, un groupe cyano, un groupe thiol, un groupe carboxyle, un groupe alkyle, un groupe monohydroxyalkyle, un groupe polyhydroxyalkyle, un groupe thioalkyle, un groupe cyanoalkyle, un groupe alcoxy, un groupe acyle, un groupe acétyloxy, un groupe cycloalkyle, un groupe aryle, un groupe alkylaryle, et des groupes de type $-NHR^W$ dans lesquels $R^W$ représente l'hydrogène, un groupe alkyle, un groupe monohydroxyalkyle, un groupe polyhydroxyalkyle, un groupe acétyle, un groupe uréido, et deux restes voisins parmi $R^5$, $R^6$ et $R^7$ peuvent former des noyaux cycliques de cinq à sept termes avec les atomes de carbone aromatiques qui les relient à l'hétérocycle, et

$R^8$ la signification de $R^2$, $R^8$ et $R^2$ pouvant être identiques ou différents,

et dans lequel

Q n'est pas relié à un atome de carbone d'un carbonyle,

V représente au moins un groupe $V^1$ et au moins un groupe $V^2$, dans lequel

$V^2$ est choisi parmi des restes hydrocarbonés divalents ou trivalents, à chaîne droite, cyclique ou ramifiée, saturés, insaturés ou aromatiques comprenant jusqu'à 1000 atomes de carbone (les atomes de carbone du reste polysiloxane $Z^2$ défini ci-dessous n'étant pas inclus dans le décompte) qui peuvent contenir le cas échéant un ou plusieurs groupes choisis parmi

-O-, -CONH-,

$-CONR^2-$, où $R^2$ est tel que défini ci-dessus,

-C(O)- et -C(S)-, et

le reste $V^2$ peut être substitué le cas échéant par un ou plusieurs groupes hydroxyle, et

le reste $V^2$ contient au moins un groupe $-Z^2-$ de formule

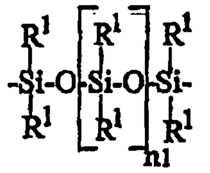

dans laquelle

les $R^1$ peuvent être identiques ou différents et sont choisis dans le groupe constitué par un alkyle en $C_1$ à $C_{22}$, un fluoroalkyle en $C_1$ à $C_{10}$ et un aryle en $C_6$ à $C_{10}$, et

$n_1$ vaut de 20 à 1000,

$V^1$ est choisi parmi des restes hydrocarbonés divalents ou trivalents, à chaîne droite, cyclique ou ramifiée, saturés, insaturés ou aromatiques comprenant jusqu'à 1000 atomes de carbone, qui peuvent contenir le cas échéant un ou plusieurs groupes choisis parmi

-O-, -CONH-,

$-CONR^2-$, où $R^2$ est tel que défini ci-dessus, les groupes $R^2$ dans les groupes $V^1$ et $V^2$ pouvant être identiques ou différents,

-C(O)-, -C(S)- et $-Z^1-$, où $-Z^1-$ est un groupe de formule

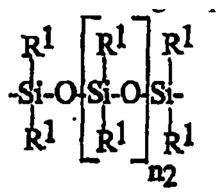

dans laquelle

$R^1$ est tel que défini ci-dessus, les groupes $R^1$ dans les groupes $V^1$ et $V^2$ pouvant être identiques ou différents, et

$n_2$ vaut de 0 à 19,

et le reste $V^1$ peut être substitué le cas échéant par un ou plusieurs groupes hydroxyle,
étant précisé que

- le reste $V^1$ ne peut pas présenter de groupe(s) ester -C(O)-O- ou -O-C(O)-,
- les restes Q trivalents et les restes $V^1$ ou $V^2$ trivalents servent exclusivement à la saturation réciproque à l'intérieur de la chaîne principale linéaire des copolymères de polysiloxane précités, et
- dans le copolymère de polysiloxane précité, le rapport molaire $V^2/V^1$ est $\neq 1$,

et dans lequel les charges positives résultant des groupes ammonio sont neutralisées par des anions d'acides organiques ou inorganiques,
ainsi que leurs sels d'addition acide.

**2.** Copolymères linéaires de polyammonium et de polysiloxane selon la revendication 1, dans lesquels $V^2$ est un groupe de formule

$$-V^{2*}-Z^2-V^{2*}-$$

dans laquelle $Z^2$ est tel que défini ci-dessus et $V^{2*}$ représente un reste hydrocarboné divalent à chaîne droite, cyclique ou ramifiée, saturé, insaturé ou aromatique comprenant jusqu'à 40 atomes de carbone, qui peut contenir le cas échéant un ou plusieurs groupes choisis parmi -O-, - CONH-, -CONR$^2$-, où $R^2$ est tel que défini ci-dessus, -C(O)- et -C(S)-, et le reste $V^{2*}$ peut être substitué le cas échéant par un ou plusieurs groupes hydroxyle.

**3.** Copolymères linéaires de polyammonium et de polysiloxane selon la revendication 1 ou la revendication 2, dans lesquels le groupe $V^1$ est choisi parmi des restes hydrocarbonés divalents, à chaîne droite, cyclique ou ramifiée, saturés, insaturés ou aromatiques comprenant jusqu'à 600 atomes de carbone, qui peuvent contenir le cas échéant un ou plusieurs groupes choisis parmi - O-, -CONH-, -CONR$^2$-, où $R^2$ est tel que défini ci-dessus, -C(O)-, -C(S)- et -Z$^1$-, où -Z$^1$- est un groupe de formule

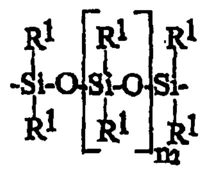

dans laquelle $R^1$ est un alkyle en $C_1$ à $C_3$, un fluoroalkyle en $C_3$ à $C_6$ ou un aryle en $C_6$, et $n_2$ est tel que défini ci-dessus.

**4.** Copolymères linéaires de polyammonium et de polysiloxane selon l'une des revendications 1 à 3, dans lesquels le rapport molaire $V^2/V^1$ satisfait la relation

$$V^2/V^1<1.$$

**5.** Copolymères linéaires de polyammonium et de polysiloxane selon l'une des revendications 1 à 4, dans lequel le rapport molaire $V^2/V^1$ satisfait la relation

$$0,0005 < V^2/V^1 < 0,9$$

**6.** Procédé de production des copolymères linéaires de polyammonium et de polysiloxane selon l'une des revendications 1 à 5, dans lequel

a) au moins un composé amine, choisi parmi un composé diamine et/ou un composé monoamine primaire ou secondaire, est mis à réagir avec au moins deux composés organiques difonctionnels aptes à réagir avec les fonctions amino du composé amine, le rapport molaire des composés organiques étant choisi de manière à satisfaire la condition $V^2/V^1 \neq 1$,
b) au moins deux moles d'un composé amine, choisi parmi un composé diamine et/ou un composé monoamine primaire ou secondaire, sont mises à réagir avec une mole d'un composé organique difonctionnel apte à réagir avec les fonctions amino du composé amine en formant un composé diamine (monomère) qui est ensuite mis

à réagir avec au moins un composé amine, choisi parmi un composé diamine et/ou un composé monoamine primaire ou secondaire, et avec au moins un autre composé organique difonctionnel apte à réagir avec les fonctions amino des composés amines,
c) un composé amine, choisi parmi un composé diamine et/ou un composé monoamine primaire ou secondaire, est mis à réagir avec un composé organique difonctionnel apte à réagir avec les fonctions amino des composés amines en formant un composé diamine (oligomère aminoterminé) qui est ensuite mis à réagir avec au moins un composé organique difonctionnel apte à réagir avec les fonctions amino des composés diamines,
d) un composé amine, choisi parmi un composé diamine et/ou un composé monoamine primaire ou secondaire, est mis à réagir avec un composé organique difonctionnel apte à réagir avec les fonctions amino des composés amines en formant un composé difonctionnel apte à réagir avec des fonctions amino (oligomère difonctionnel) qui est ensuite mis à réagir avec au moins un composé amine, choisi parmi un composé diamine et/ou un composé monoamine primaire ou secondaire, et au moins un autre composé apte à réagir avec des fonctions amino,

dans lequel des monoamines monofonctionnelles, de préférence tertiaires, ou des monoamines appropriées inaptes à prolonger la chaîne et/ou des composés monofonctionnels aptes à réagir avec des fonctions amino peuvent le cas échéant être ajoutés à la réaction comme agents de rupture de chaîne et la stoechiométrie des fonctions amino et des groupes fonctionnels aptes à réagir avec des fonctions amino dans la dernière étape de la réaction est toujours sensiblement égale à 1:1, et
dans lequel les fonctions amino éventuellement présentes peuvent être protonées, alkylées ou quaternisées.

**7.** Procédé selon la revendication 6, dans lequel les groupes fonctionnels des composés difonctionnels aptes à réagir avec des fonctions amino sont choisis dans le groupe constitué par des groupes époxy et des groupes haloalkyle.

**8.** Utilisation des copolymères linéaires de polyammonium et de polysiloxane selon l'une des revendications 1 à 5 ainsi que des copolymères linéaires de polyammonium et de polysiloxane obtenus suivant la revendication 6 ou la revendication 7 dans des formulations cosmétiques, dans des détergents ou pour le traitement de surface de substrats.

**9.** Utilisation selon la revendication 8 pour traiter ou apprêter des fibres.

**10.** Compositions contenant au moins un copolymère linéaire de polyammonium et de polysiloxane selon l'une quelconque des revendications 1 à 5 ou l'un au moins des copolymères linéaires de polyammonium et de polysiloxane obtenus suivants l'une des revendications 6 ou 7 ensemble avec au moins un autre constituant habituel pour la composition.

**11.** Composition selon la revendication 10, qui est une composition de détergent ou une composition cosmétique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 441530 A
- US 5591880 A
- US 5650529 A
- US 5807956 A
- US 5981681 A
- US 5602224 A
- US 5098979 A
- US 5153294 A
- US 5166297 A
- US 6242554 B
- DE 3719086 C
- EP 282720 A
- US 6240929 B
- WO 0210259 A
- WO 0071806 A
- WO 0071807 A
- US 6211139 B
- WO 9914300 A
- DE 3236466 A
- WO 0210257 A
- DE 4318536 A


### In der Beschreibung aufgeführte Nicht-Patentliteratur


- **J.V.GRUBER.** Colloids and Surfaces B. *Biointerfaces,* 2000, vol. 19, 127-135
- Silicone, Chemie und Technologie. Vulkan-Verlag, 1989, 82-84
- **B. MARCINIEC.** Comprehensive Handbook on Hydrosilylation. Pergamon Press, 1992, 122-124
- **B. MARCINIEC.** Comprehensive Handbook on Hydrosilylation. Pergamon Press, 1992, 116-121127-130134-137151-155
- 17. Organikum, Organischchemisches Grundpraktikum. VEB Deutscher Verlag der Wissenschaften, 1988, 189-190